# EUROPEAN PATENT SPECIFICATION

(11) **EP 4 626 371 B1**
(45) Date of publication and mention of the grant of the patent: **02.12.2026**
(21) Application number: 23734032.8
(22) Date of filing: 24.05.2023
(51) Int. Cl.: A61F 5/00, A61F 5/058

(54) **INDIVIDUALIZED SKELETAL SUPPORT WREATH FOR EXTRAORAL ORTHOPEDIC DEVICE FOR JAW PROTRACTION AND COMPUTER METHOD AND PROGRAM FOR DESIGNING THE SAME**
INDIVIDUELL ANGEPASSTER SKELETTSTÜTZKRANZ FÜR EINE EXTRAORALE ORTHOPÄDISCHE VORRICHTUNG ZUR KIEFERPROTRAKTION SOWIE COMPUTERVERFAHREN UND PROGRAMM ZU DESSEN KONSTRUKTION
COURONNE DE SOUTIEN SQUELETTIQUE PERSONNALISÉE POUR DISPOSITIF ORTHOPÉDIQUE EXTRA-ORAL DESTINÉ À LA PROTRACTION DE LA MACHOIRE, ET PROCÉDÉ INFORMATIQUE ET PROGRAMME POUR SA CONCEPTION

(30) Priority: 28.11.2022 GR 20220100971
(43) Date of publication of application: 08.10.2025
(73) Proprietor: Koutzoglou, Stylianos, 74132 Rethymno (GR); Koutzoglou, Eleni, 74132 Rethymno (GR); Koutzoglou, Despoina, 74132 Rethymno (GR)
(72) Inventor: Koutzoglou, Stylianos, 74132 Rethymno (GR); Koutzoglou, Eleni, 74132 Rethymno (GR); Koutzoglou, Despoina, 74132 Rethymno (GR)
(74) Representative: Banteka, Ioanna
(86) International application number: PCT/GR2023/000022
(87) International publication number: WO 2024/115930

(56) References cited:
- WO-A1-2021/090035
- US-A- 2 325 300
- US-A1- 2004 199 094
- US-A1- 2012 296 249
- US-A1- 2021 045 844

## Description

This invention relates to the field of extraoral devices for the direct protraction of the maxilla through forward, downward or upward adjustments, as each case requires, as well as for the direct or indirect protraction of the mandible, where it is necessary.

Through this extraoral device, direct traction is applied to the maxilla immediately after its "rapid expansion" [1-4] using the traditional or alternate way [5-7], with elastic bands which are attached to the extraoral device and some classic mechanisms of "rapid maxillary expansion", as in: [1-4] or through skeletal support, as in: [8]. The mandible can also be protracted both directly or indirectly in patients with a skeletal Class II malposition of the jaws, when the extraoral device is combined with intraoral mechanisms in the maxilla and/or in the mandible.
**A.** Until now, the commonly used extraoral devices for the protraction of the maxilla, are the "facemasks" designed by Delaire [9, 10] and Pettit [11] which are related only to the skeletal Class III malocclusion. In both of these devices, the forehead and chin are used as support in order to apply elastic forces, which currently amount to approximately 400 gr per side. In this way, the protraction of the maxilla is achieved (action), while the simultaneous pressure (reaction) on the temporomandibular joint tissues (particularly on the condyle, articular disc and fossa) could lead to a TMJ derangement in patients predisposed to that. Extraoral orthopedic devices used in the same way (support on the frontal bone and the chin) are the Turley "mask" [12] and the Face Mask/Reverse -pull Headgear Tübinger Model [13]. The "Sky Hook" headgear [14], which is supported on the cranial bones, parietal and occipital, as well as on the chin, is also used for the protraction of the maxilla after its "rapid expansion". The elastic forces applied to the maxilla originate mostly from its chin support.
**B.** The Grummons facemask [15] uses the forehead as support and, instead of the chin, the infraorbital zygomatic area. This type of support differentiates this device from all of the above-mentioned. The face mask produced by the Leone company [16] uses the same support-area. While these devices are used with the aim to protract the maxilla after its rapid expansion, the therapy results are highly controversial because of the use of the infraorbital zygomatic support areas. These areas of the face comprise the zygomaticomaxillary sutures, which are subjected to a reactive pressure, thus generating the maxillary pulling action.
**C.** From time to time, devices have appeared for the maxillary protraction which use as skeletal anchorage only the frontal bone of the skull. The "maxillary modified protraction headgear" [17] is one of them. The only advantage of this device is its support. The non-use of the mandible, as support for the protraction of the maxilla, eliminates the possible sideeffect of TMJ derangement. The industrial production, as well as the practical use of this device, are enormously impeded by the personalized bending of the extraoral and intraoral wires separately in each patient, aiming, firstly, at an easy insertion of the intraoral wires to the tubes of a concrete intraoral appliance and secondly, at avoiding the well-known side-effects during maxillary protraction.
D. Nowadays, intraoral orthopedic devices of skeletal support are widely used in the treatment of Angle Class III malocclusion. Mini-plates [18] or titanium mini-plates [8] are surgically attached to the infra-zygomatic region in the maxilla and between the permanent canines and lateral incisors in the mandible [18] or underneath the permanent lower incisor apices [8]. The skeletal anchorage in the maxilla could also comprise apart from teeth two palatal mini-screws [8], which are incorporated into an appliance (Hybrid-hyrax RME) used for "rapid maxillary expansion" and protraction of the maxilla, or could be titanium miniplates placed on the infra-zygomatic crest above the buccal roots of the first permanent molar [18]. Growth repression of the mandible occurs because of the simultaneous backward compression of the chin from these devices. The glenoid fossa is transformed with a corresponding displacement of the condyle [19]. These anatomic changes are not required in all Angle Class III malocclusion cases, such as in patients who present a physiological growth in their mandible in conjunction with maxillary retrognathism due to the size or position of the maxilla [20]. This is commonly the case in cleft lip and palate patients. In the cases where these anatomic changes could be a positive outcome, their medical usefulness is lost because of the compression of the TMJ tissues, which could lead to a TMJ dysfunction. Extraoral orthopedic devices supported on the frontal bone and the chin, in combination with titanium infra-zygomatic mini-plates, have been used for the maxillary protraction [21]. In all these three types of devices in category D, the mandible is pushed backwards, while the maxilla is protracted. In addition to the risk of creating a TMJ derangement, as already mentioned above (category A devices), there are a lot of cases in which the mandible is positioned properly in the face. Nevertheless, all these types of devices operate in a compensatory manner, pushing the mandible backwards, modifying its growth and influencing its function and the aesthetic of the face adversely.
   Functionally, besides the side-effects that can develop from the compression of the TMJs, it should be pointed out that the backward push of the mandible, followed by the tongue, could contribute to future problems with sleep apnea. Not forgetting, that all orthodontic appliances, which are used in the therapy of sleep apnea, are designed to bring the mandible mainly forward, with the tongue following this displacement of the mandible with simultaneous relief of the airway on the oropharynx level.
**E.** The "orthodontic appliance" [22] [U.S. PATENT: 5,158,451], the "Mandibular advancement" [23] [U.S. PATENT 2012/0040301 A1], the "orthopedic device for the protraction of the maxillary arc" [24] [WO2017089971 (A1)], the "maxillary protraction device" [25] [US2018028282 (A1)] and the "extraoral orthopedic device for the direct protraction of the maxilla and the indirect protraction of the mandible" [26] [WO2021090035A1] are specifically designed so that in use they avoid TMJ compression and increase the volume of the oral cavity.

US2004199094A1 shows a craniomaxillofacial distraction device including a distraction component which applies distracting forces to treat craniofacial anomalies. The distraction component is mounted onto a support which is connected to a helmet. The helmet secures the distraction device to the head of a patient.

U2325300A shows an appliance for use in the treatment of fractures, malformations of the jaws and contiguous bone structures, comprising a headband having an underlying band and an overlying band having a series of perforations therein through which the ends of a wire are adapted to be inserted and between which bands said ends are adapted to be maintained.

WO2021090035A1 shows an extraoral device for direct protraction of the maxilla and indirect protraction of the mandible, ergonomically designed for its fast and accurate placement, comprising a circular cranial support, perpendicular girders by which, the circular cranial support is conjugated with a horizontal girder, which comprises screws to mount elastic tractions between an intraoral device of "rapid palatal expansion" and the extraoral device, aiming to the forward movement of the maxilla. In conjunction with this device, another intraoral device in the mandible is used, in the case of its indirect protraction.

US2021045844A1 shows a headgear can be worn on a user's head to adjustably fix a mouthpiece inserted in an oral cavity. The headgear includes: a front pad brought into contact with and pressed against a front pressure point of an outer surface of the buccinator between the zygomatic bone and the mandible in the face region; one or more rear pads brought into contact with and pressed against one or more rear pressure points so that a resultant force becomes a reaction force paired with a pressing force of the front pad, in a region from the top to the back of the head; a frame fixing the front pad and the rear pads; a front horizontal bar fixed to the frame at a position in front of the lip region of the head; and an adjustment part installed on the front horizontal bar to mount the mouthpiece.

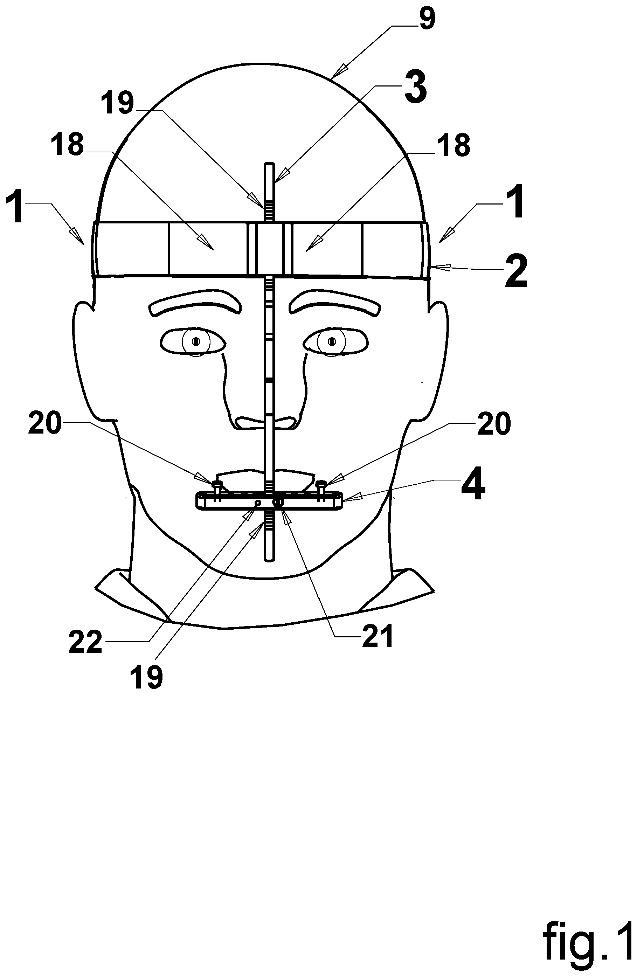

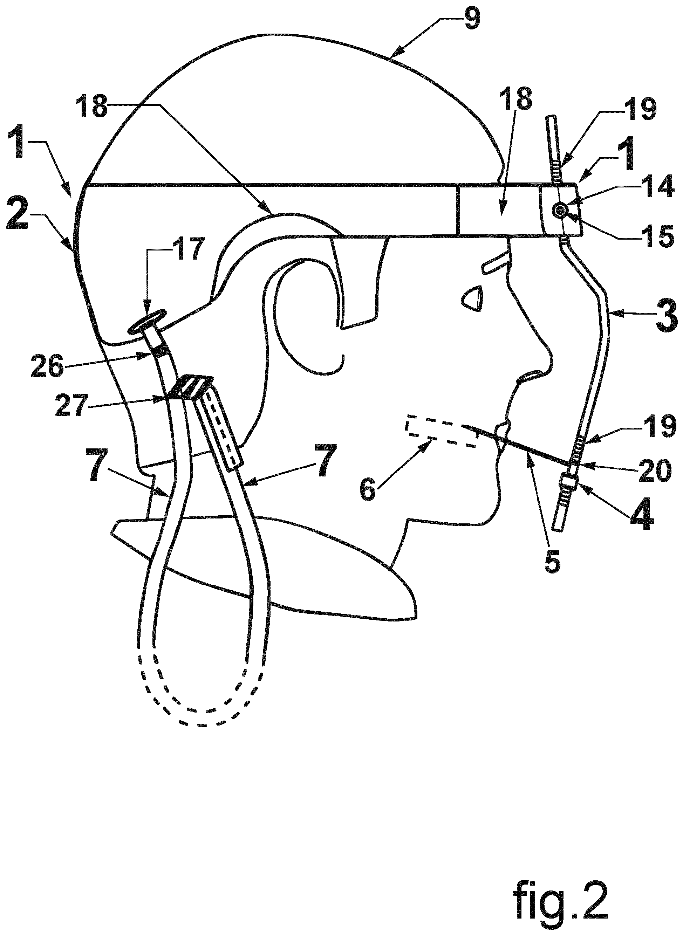

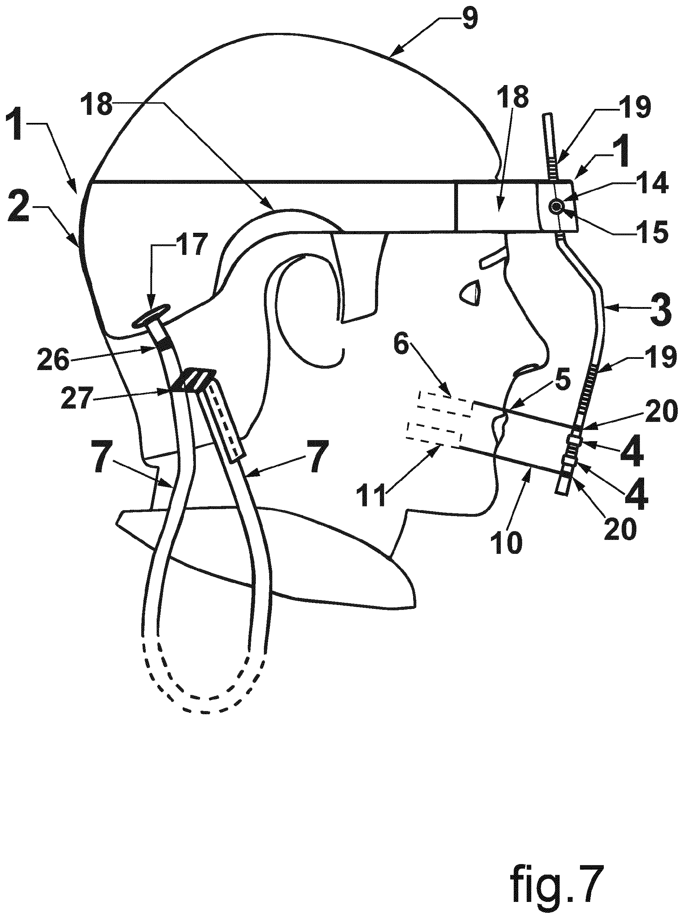

The device described in figures 1-16, provides excellent stability, greater efficiency, ergonomics and flexibility in its application and use, due to:
**1.** The true anatomical design of the skeletal support wreath on the neurocranium and the perfect stabilization of the wreath on it because of its excellent fit and through the two elastic fixation straps (7) of the same length, passing under the axillae and around the shoulders reconnecting with their cranial ends, both of which pull and immobilize the skeletal support wreath downwards, in order to avoid its dislodgment when the device (1) is in use.
**2.** The construction material of the connecting and the mouth girders is an aluminum alloy. Using this material, the girders are both very light and resistant to corrosion, especially through their anodizing. The aluminum alloy is a nickel-free metal, thus avoiding nickel skin allergy. It is also emphasized that due to their fixed design, the metal girders of the device described herein are removable/replaceable and can be reused, after sterilization, with any other patient, in the context of circular economy, something that to date has not been incorporated into any of the existing extraoral orthopedic devices with a similar purpose.
**3.** This device and the orthopedic device [26] follow the same practice: neither pushes the mandible backwards in order to move the maxilla forwards. In addition, this device consists of one connecting girder (3, fig.1) in the middle of the face instead of two on the sides, as in [26]. Because of that, the patient can also sleep on their side, which cannot be the case using the device [26].
**4.** Another advantage of this device in relation to [26] is that there is no need to have in its skeletal support wreath a guided-adjustable-tightening mechanism because of its manufacturing process (scanning of the head - digital creation and 3D-printing of a fully individualized wreath of exceptional accuracy). In this way, it is simpler, more comfortable and much more stable, because it is complemented by the elastic fixation straps (fig.2, 4, 7). This prevents discomfort on the forehead and generally on the patient's head, which is vital for the patient's cooperation. This provides ease of application, stability and above all optimal comfort.
**5.** The direct protraction of the maxilla, mainly in patients who present opisthognathic maxilla in the face (maxillary retrognathia, maxillary deficiency), regardless of their Angle Class I or Class II dental relationship, is not referred to in the above-mentioned citations of category E, except in [26]. In these malocclusions, the protracted maxilla can be held in its new position by this device and after that, the mandible can be moved forward by using another intraoral mechanism. Nevertheless, the mandible could also be protracted simultaneously by using a second mouth girder (4, fig.7) incorporated into the connecting girder (3), but inserted upside down. This is the only way the aetiologic therapy of the maxillary retrognathia in Angle Class I and II malocclusions can be achieved.
**6.** A further very significant disadvantage both of [26] and of all the above-mentioned devices of category E, except in [25], is the risk of dislodgment of the skeletal support wreath, due to the upward and forward torque created during their use. Through the design of the herein described device this risk is minimized, because it is based on two key features: **a.** The methods used in the manufacture of the skeletal support wreath, described below, ensure an excellent anatomical fit on the patient's head, something that is not provided in any of the above-mentioned devices in all categories, and which is necessary for its stable hold on the neurocranium, but also for the comfort of the patient when they wear something that is individualized made to their measurements. **b.** Due to the elastic fixation straps (7) that are attached to the skeletal support wreath, the risk of its dislodgment is minimal, regardless of the magnitude of the forces aimed at the protraction of the maxilla or both jaws.

No matter how well the skeletal support wreath fits, there are heads of patients with such anatomy (small area on the dorsal surface of the parietal bones and occipital bone) and/or patients with oily and fine hair, that no matter how fast the tightening mechanism [26] is in the frontal bone area, there is always the risk of dislodgment. This is due to the upward and forward torque of the dorsal portion of the ellipsoidal skeletal support wreath in response to the application of the force of elastic tractions to protract the maxilla. An opposite force must be exerted on the dorsal part of the wreath, which is achieved through the suspension of two elastic fixation straps (7, fig.2, 4, 7) attached to this part. In this way, the skeletal wreath can be worn on any human skull and be used safely in the exercise of any force of direct traction of the maxilla and direct or indirect of the mandible, without the slightest risk of dislodgment.

The tightening mechanism in the frontal bone of [26] device, which is of vital importance, because of its excessive tightening, in order to avoid the dislodgment of the wreath, could create such pain in patients, that, in turn, drastically reduces the patient's motivation to cooperate.

**7.** Due to the methods of design and manufacture of the herein-presented skeletal support wreath of cranial form, described below, which lead to an excellent anatomical fit and a precise placement of its all individual parts, it is manufactured finally with very high accuracy, which is not the case in any one of the existent devices [22-26].

**8.** Finally, as mentioned in the design of [26], the part of the skeletal support wreath directly distal from the cylindrical slots, which accommodates the connecting cylindrical girders, "becomes extremely thin", which, we found, could lead to a failure of the skeletal support wreath in these areas, because these parts of the wreath receive an exceptional load during the operation of the device, proportional to the traction forces on the maxilla. The herein-described device boasts reinforcement webs (18), i.e. anti-bend supports, configured to withstand the actions applied during use.

Today, in patients with Angle Class II malocclusion, many of whom are in a growth phase and their growth potential can be modified, the orthodontist uses such techniques to move the permanent maxillary molars distally, aiming at an Angle Class I molar relationship (only dental and not skeletal - aetiological approach), ignoring very often the mandibular retrognathia. In the best cases, the orthodontist uses functional appliances, removable, fixed or hybrid types, to move the mandible forward. Even in these cases, the devices used are supported in the maxilla to reposition the mandible forward, which results in suppressing forward maxillary growth (when there is action, there is reaction).

In Angle Class II malocclusion cases, in which the maxilla is located in a harmonic or in a retrognathic position within the face, which is also apparent from the increased nasolabial angle in the profile of the patient, the same therapy techniques are used, aiming at an Angle Class I dental relationship, ignoring the skeletal data of the jaws, as parts of the face in its entirety. In order to create harmonic and juvenile relationships of the two jaw bones within the face, orthofacial surgical interventions are used in adults, moving both the maxilla and the mandible forwards and secondarily creating an Angle Class I molar relationship [27].

In cases where the original location of the maxilla within the face is in a prognathic position (severe labial inclination of the upper permanent incisors, very reduced nasolabial angle) or in the true skeletal Class III cases, in which the mandible is prognathic because of its size with respect to the cranial base [28], we accept the use of the widely-used devices and techniques of today, for example in the true skeletal Class III cases, the compensatory support in the mandible and compression of the temporomandibular joints, in an effort to prevent the patient from orthognathic surgery in the future, but with possible negative implications.

The invention relates to a convenient extraoral orthopedic mechanism, stable and precise in its adjustment and function, which in combination with an intraoral device is able to protract the maxilla directly, easily and without side-effects, as well as the mandible, directly or indirectly, mainly in cases of skeletal Class III and Class II malocclusions respectively. This mechanism can be used in cooperative, growing young patients.

The object of the invention is defined by the computer implemented method of designing an individualized skeletal support wreath as defined in claim 1, a computer program as defined in claim 5, an individualized skeletal support wreath as defined in claims 6 and an external orthopaedic device as defined in claim 7.

Nowadays, in an Angle Class II malocclusion patient, it is impossible for an orthodontist to firstly reposition the maxilla further forward and deteriorate temporarily the dental Class II relationship, then reposition the mandible much further forward, holding simultaneously the maxilla in its protracted position, even with our suggested extraoral orthopedic device. Indeed, there is a plethora of growing young patients displaying an Angle Class II malocclusion, with both jaw bones in a retrognathic position whose facial growth can be modified. Nevertheless, the orthodontist "going with the flow" holds the maxilla in its original retrognathic location within the face and, with its support, tries to relocate the mandible forwards.

The proper head and body posture, unimpeded nasal breathing, mastication of hard as well as soft food and physiological swallow pattern should be very seriously considered, in the context of holistic orthodontic treatment.

This extraoral orthopedic device can be especially used in cooperative growing young patients, for whom the modification of the growth of their stomatognathic system is possible, for: **a.** The therapy of skeletal Class III malocclusion, protracting the maxilla directly after its "rapid expansion" [1-4, 8] without the simultaneous compression and impediment of mandible growth, thus avoiding: probable dysfunction of the TMJs, reduction of the tongue's vital space, which could lead, in turn, to possible sleep apnea and other problems. We especially mention the treatment of patients suffering from cleft lip and palate, in which the maxilla is retrognathic due to the connective tissue scars on the upper lip and upper jaw created by orthognathic/plastic surgery in the upper lip and maxilla. However, following the usual practice, the orthodontist uses a common physiologic mandible in size and position as support to protract a retrognathic maxilla [29]. Invariably in these cases, due to actionreaction, the mandible is moved backwards resulting in what has been explained above. **b.** The therapy of skeletal Class II malocclusion, with Angle Class I or II dental relationship, in the cases where the maxilla is retrognathic in its original position within the face. After the activation of viscerocranium sutures using the "rapid maxillary expansion" technique [1-4, 8] in the traditional way, the maxilla is protracted, initially, and subsequently held in its relocated position by the extraoral device. Finally, the mandible is directly or indirectly protracted using an additional intraoral mechanism. In this way, the growth modification of both maxilla and mandible gives an unmatched aesthetic result to the appearance of the whole face and greatly helps the function of breathing and tongue after the space in the mouth cavity has significantly increased. So, the probable cause of sleep apnea is notably reduced.

### DESCRIPTION OF DRAWINGS

The extraoral orthopedic device **(1),** as illustrated in detail in figures 1-16, is basically composed of 4 parts: **the skeletal support wreath (2)** on the neurocranium (9), **the connecting girder (3), the mouth girder (4),** the traction means, such as elastic bands (5, 10), through which the device is coupled with an intraoral mechanism of "rapid maxillary expansion" (6) or with another intraoral mechanism fixed in the mandible (11) and **the elastic fixation straps (7),** passing through the axillae with firm contact and around the shoulders reconnecting with their cranial ends, both of which pull and immobilize the skeletal support wreath downwards, in order to avoid its dislodgment when the device **(1)** is in use.
**Figure 1** schematically illustrates: the frontal view of the device (1), the wreath (2) attached to the neurocranium (9), the connecting girder (3) in the middle of the face and the mouth girder (4). On the mouth girder are illustrated the elongated elements with a mounting means, such as the mounting screws (20), the anterior fixing screw (21) for the immobilization of the mouth girder and the threaded hole (22) for the posterior fixing screw (21) responsible also for the immobilization of the mouth girder. The markings (19) on the cranial and caudal parts of the connecting girder (3) are also illustrated.
**Figure 2** schematically depicts: the profile of the device (1) in use, the wreath (2) on the neurocranium (9), the connecting girder (3) with its markings (19), the mouth girder (4), the traction means (5), attached extraorally to the mounting screws (20) and intraorally to a mechanism of "rapid maxillary expansion" (6) and the right hand-side elastic fixation strap (7). The right elliptical slit (17) of the wreath for the passage of the cranial end of the elastic strap and its fixation area (26), as well as the cylindrical hole (14) in the wreath guiding the fixing screw (15) for the immobilization of the connecting girder (3) are also illustrated. After the straps (7) pass through the axillae and around the shoulders, their caudal ends are reconnected with their cranial ends through the adjustable and support strap buckles (27), either in the area of the chest, as represented here, or the back, depending on their insertion direction through the elliptical slits (17), in order to adjust the distance between wreath and axillae, as well as their pulling power, which provides the required stability for the proper fixation of the wreath on the neurocranium and preventing the skeletal support wreath (2) from being dislodged during operation of the device (1). The reinforcement webs (18), i.e. anti-bend supports, in the frontal and lateral areas of the wreath, are also presented.

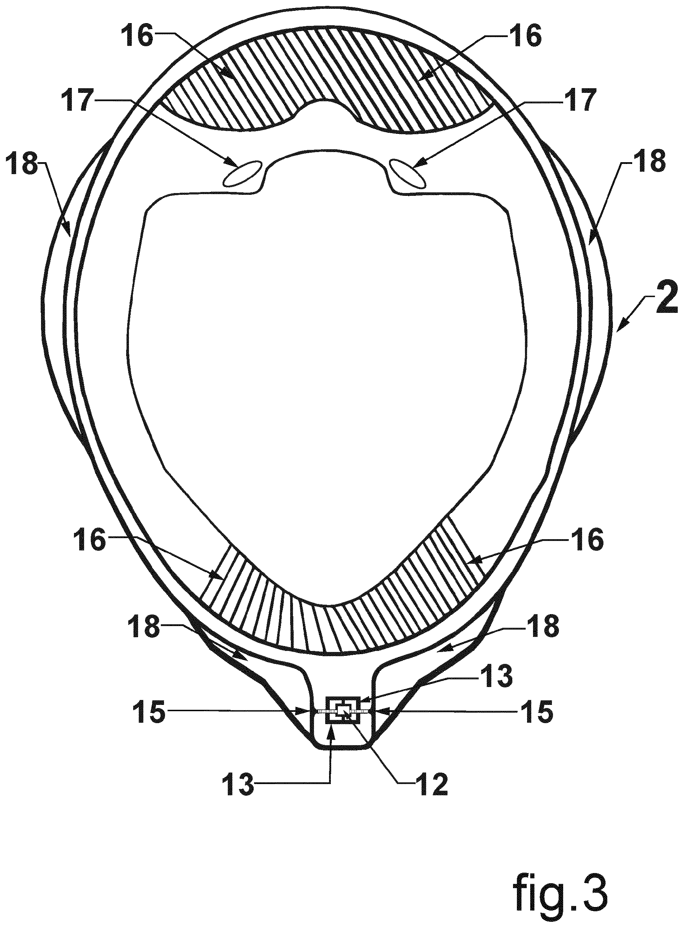

In **figure 3****,** the top view of the wreath (2) is illustrated. The metallic parts (13) create a cuboidal slot (12) responsible for the passage of the connecting girder (3). The fixing screws (15) for the immobilization of the connecting girder (3), the elliptical slits (17), the reinforcement webs (18), i.e. anti-bend supports, and the interior soft inlay (16), in the form of soft material pads, of the skeletal support wreath (2), are also illustrated. The reinforcement webs (18) prevent the bending of the wreath (2) in its frontal and lateral areas during use of the device (1).

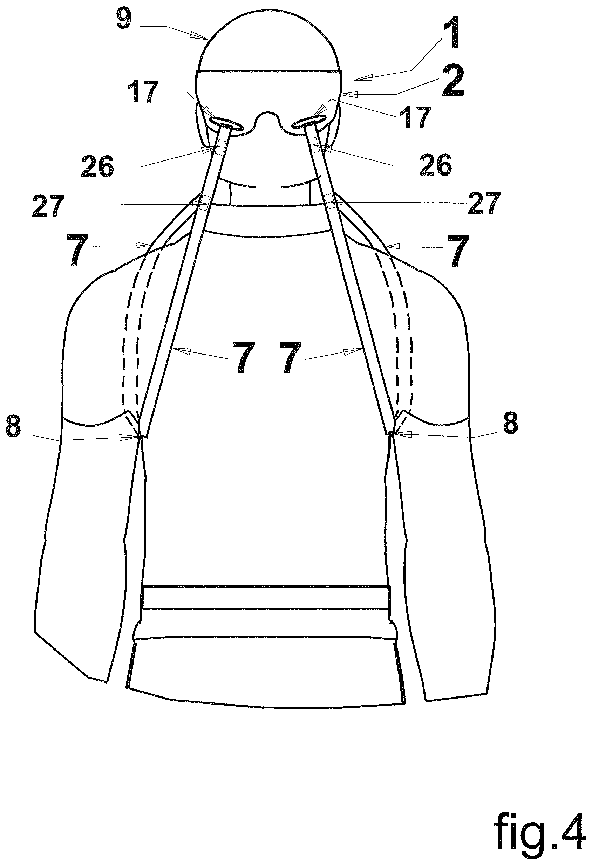

In **figure 4****,** the back of a patient and the dorsal view of the device (1) with its wreath (2) placed on the neurocranium (9) are illustrated. The elliptical slits (17) for the passage of the cranial end of the elastic straps (7) and their attachments (26), the axillae (8), through which with firm contact and around the shoulders of the patient the straps (7) pass, are also shown. After the straps (7) pass through the axillae and around the shoulders, their caudal ends are reconnected with their cranial ends through the adjustable and support strap buckles (27).

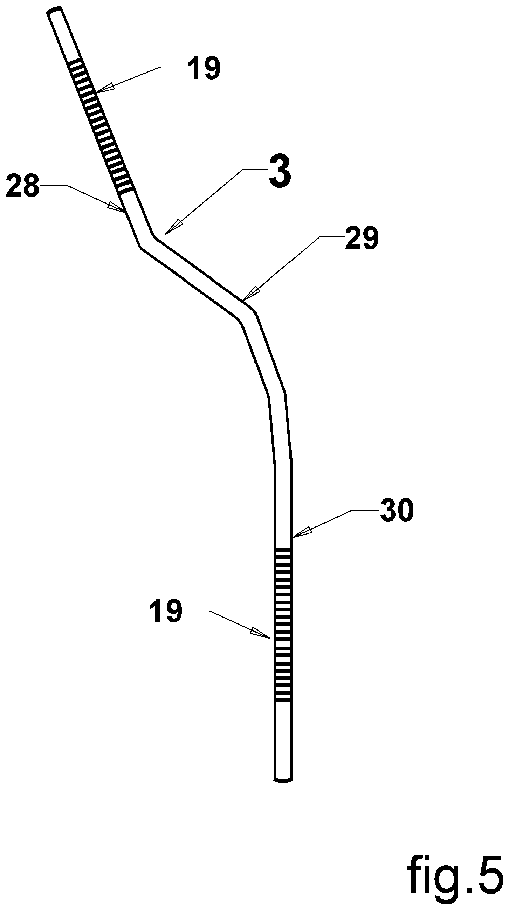

In **figure 5****,** the metallic connecting girder (3) of a rectangular cross-section, removeable and reusable after sterilization, and its markings (19) on the cranial part (28) of the connecting girder (3) responsible for an easy and accurate replacement and adjustment in its height in the wreath (2) and the markings (19) on the caudal part (30) of the connecting girder (3) responsible for an easy and accurate replacement and adjustment in the height of the mouth girder (4) along the connecting girder (3), as well as its angled middle part (29), are illustrated.

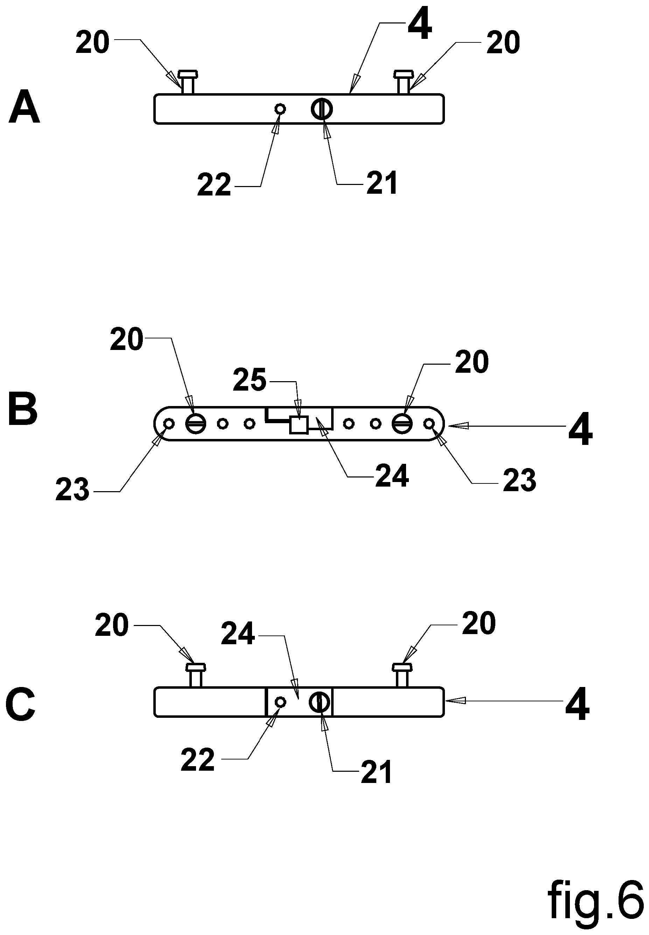

In **figure 6****,** the aluminum-alloy mouth girder (4), which is removable and reusable after sterilization, is illustrated. In **view A** of this figure, the front surface of the mouth girder at a right angle, is shown, where the elongated elements with a mounting means, such as mounting screws (20), for the traction means, such as elastic bands (5, 10), the anterior fixing screw (21) for the immobilization of the mouth girder (4) and the threaded hole (22) for the posterior fixing screw (21) responsible also for the immobilization of the mouth girder are illustrated. In **view B,** the top view of the mouth girder (4) is shown. The separate aluminum-alloy part (24), incorporated into the mouth girder through the fixing screws (21) responsible for the immobilization of the mouth girder (4), as well as the cuboidal slot (25) for the passage of the connecting girder (3) are also illustrated. The threaded holes (23) responsible for the accommodation of the mounting screws (20) are also displayed. Finally, **in view C,** the rear view of the mouth girder at a right angle is shown. The mounting screws (20) for the traction means, the separate aluminum-alloy part (24), the posterior fixing screw (21) and the threaded hole (22) for the anterior fixing screw (21), responsible also for the immobilization of the mouth girder, are illustrated.
**Figure 7** schematically depicts: the profile of the device (1) in use, the wreath (2) on the neurocranium (9) with its reinforcement webs (18), the connecting girder (3) and two mouth girders (4), one for the direct traction of the maxilla and the other placed upside down for the direct traction of the mandible. The traction means, such as elastic bands (5, 10), attached extraorally to the mounting screws (20) and intraorally to a mechanism of "rapid maxillary expansion" (6) and another intraoral mechanism (11) fixed in the mandible correspondingly, as well as the right hand-side elastic fixation strap (7), are also illustrated.

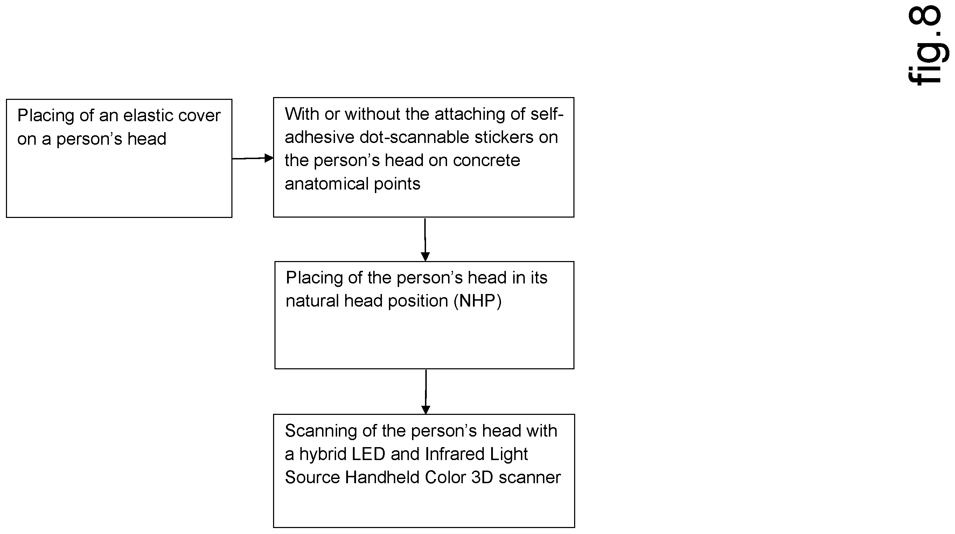

In **figure 8****,** a flowchart of the pre-scanning procedure of a person's head followed by scanning with a hybrid LED and infrared Light Source Handheld Color 3D scanner is presented.

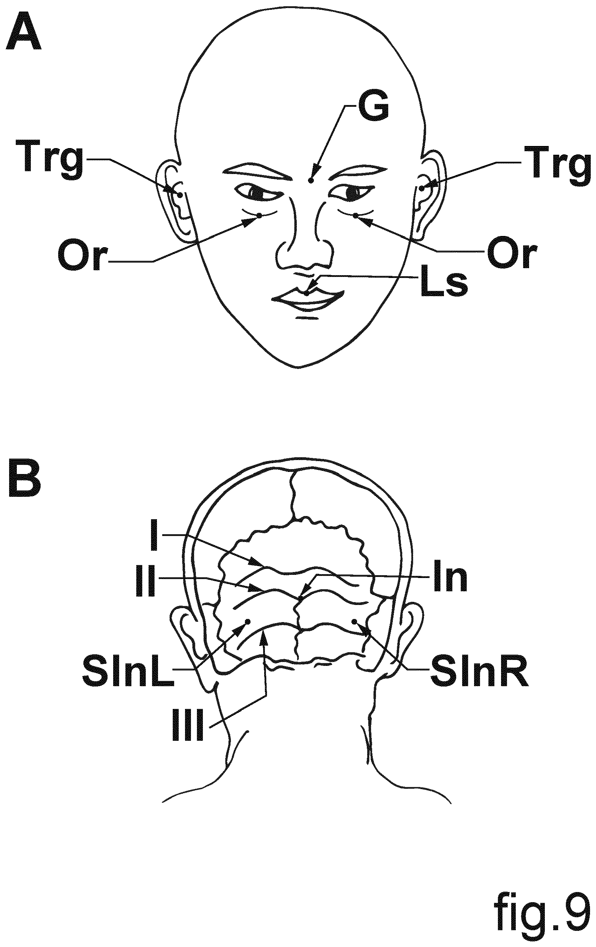

In **figure 9****,** in **view A,** the following six anatomical (craniometric) points are presented, on which self-adhesive dot-scannable stickers could be placed on the person's head before scanning: the Glabella (G), the left and right Orbitale (Or), the left and right Tragion (Trg) and the Labrale superior (Ls). In **view B,** the following three anatomical points are shown: the Inion (In, the outermost craniometric point of the external occipital protuberance) and the left and right outermost points of the prominences between the superior and inferior nuchal lines on the caudo-lateral areas of the occipital bone, the Sub-Inion left (SInL) and Sub-Inion right (SInR). The final two anatomical terms were named and abbreviated to facilitate communication. The nuchal lines of the occipital bone (supreme, I - superior, II - inferior, III), are also illustrated.

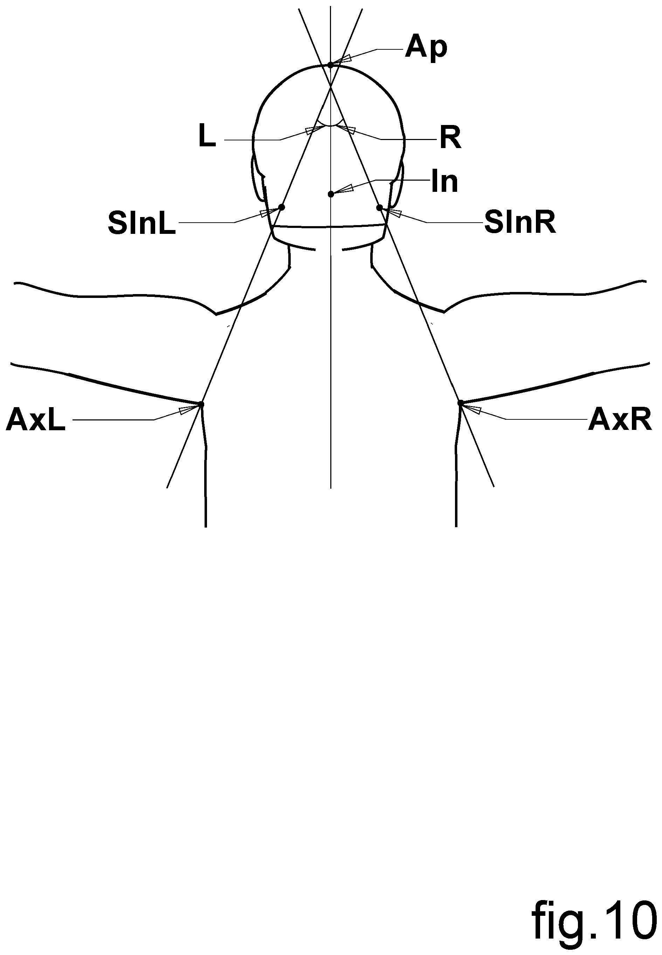

In **figure 10****,** the rear view of the trunk and the head of a person wearing a wig cap, whose arms are raised and head is positioned in its natural head position (NHP), are illustrated. The left and right axillae (abbreviated as AxL and AxR), the SInL, the SInR, the Inion (In), as well as the apex of the patient's head (abbreviated as Ap) are also presented. The line section InAp passes normally through the midsagittal plane. The extensions of the line sections AxL-SInL and AxR-SInR create the angles L and R with the line crosses the anatomical (craniometric) points In and Ap. These two angles are used in the digital design of the two elliptical slits (17), when the applied elastic fixation straps (7) pass through the axillae (fig.4).

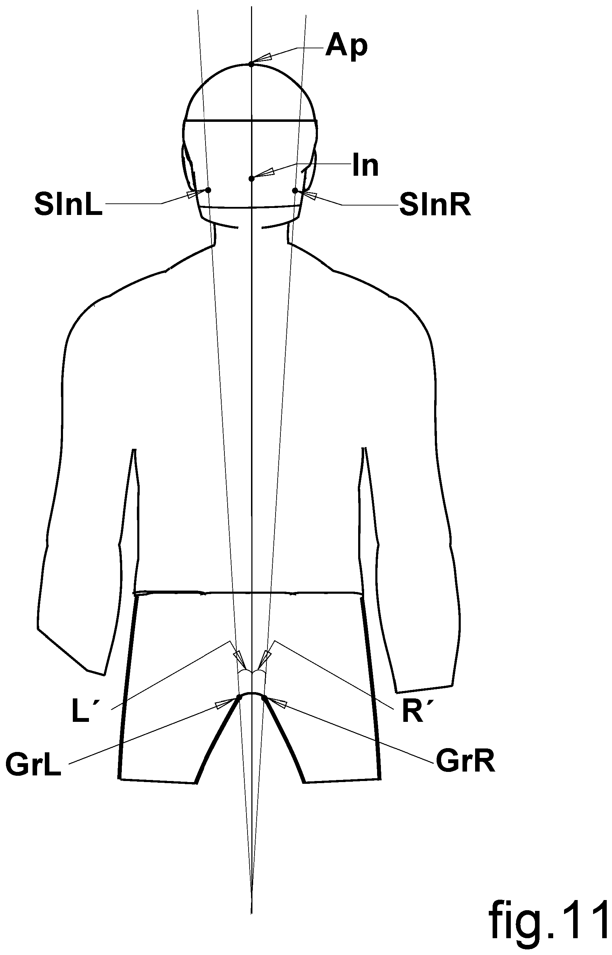

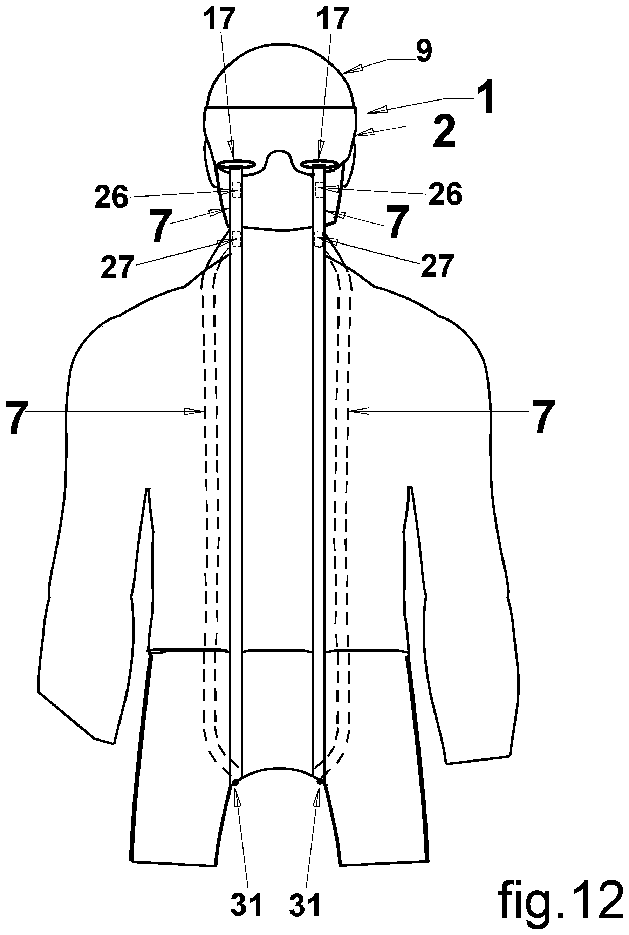

In **figure 11****,** the rear view of the body and the head of a person wearing a wig cap, whose head is positioned in its natural head position (NHP), are illustrated. The left and right groins (abbreviated as GrL and GrR), the SInL, the SInR, the Inion (In), as well as the apex of the patient's head (Ap) are also shown. The line section InAp passes normally through the midsagittal plane. The extensions of the line sections GrL-SInL and GrR-SInR create the angles L' and R' with the line crosses the anatomical points In and Ap. These two angles are used in the digital design of the two elliptical slits (17), when the applied elastic fixation straps (7) pass through the groins (fig.12).
In **figure 12****,** the rear view of the body of a patient wearing the extraoral orthopedic device (1) is depicted. The wreath (2) with its elliptical slits (17) for the passage of the cranial end of the elastic straps (7) and their attachments (26), the groins (31) of the patient, through which with firm contact and around the top of their thighs the straps (7) pass, are also illustrated. After the straps (7) pass through the groins and around the thighs, their caudal ends are reconnected with their cranial ends through the adjustable and support strap buckles (27) in the frontal area, as represented here, in order to adjust the distance between wreath and groin as well as their pulling power, which provides the required stability for the proper fixation of the wreath on the neurocranium and prevents the skeletal support wreath (2) from being dislodged during operation of the device (1).

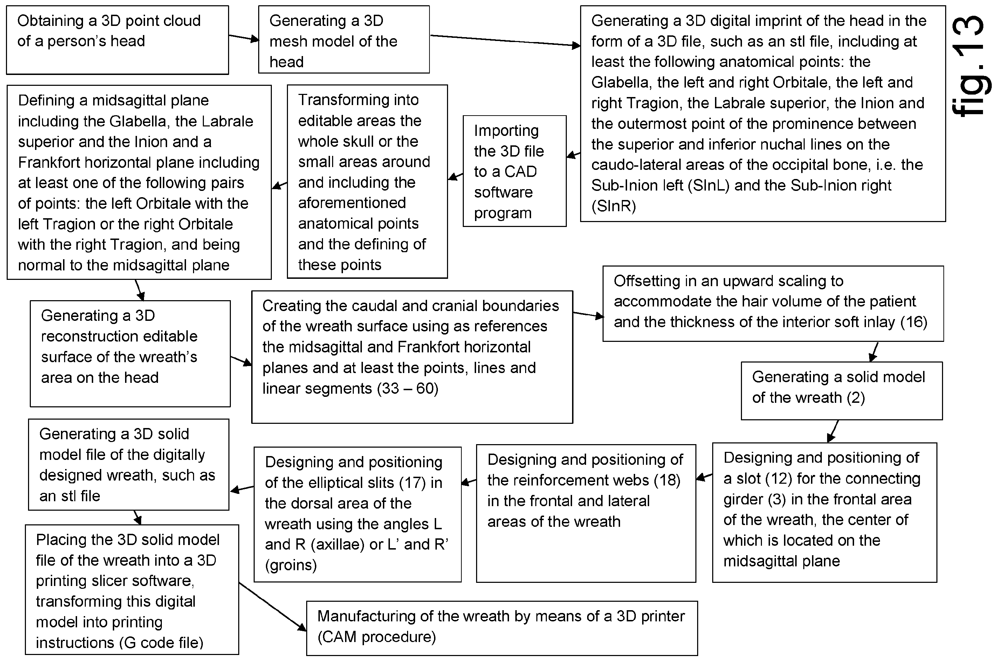

In **figure 13****,** a flowchart of the workflow of the computer-aided design and manufacturing (CAD-CAM) of the individualized skeletal support wreath (2), also comprising the digital design of the caudal and cranial boundaries of the wreath using as references the midsagittal and Frankfort horizontal planes and at least the points, lines and linear segments (33 - 60), the individualized placement of the connecting girder (3), the positioning of the reinforcement webs (18) on the wreath (2) and the elliptical slits (17), as well as the offsetting responsible for the proper adaptation to the patient's hair thickness and the thickness and quality of the interior soft inlays, after the scanning of a person's head using a structured light 3D scanner, is illustrated.

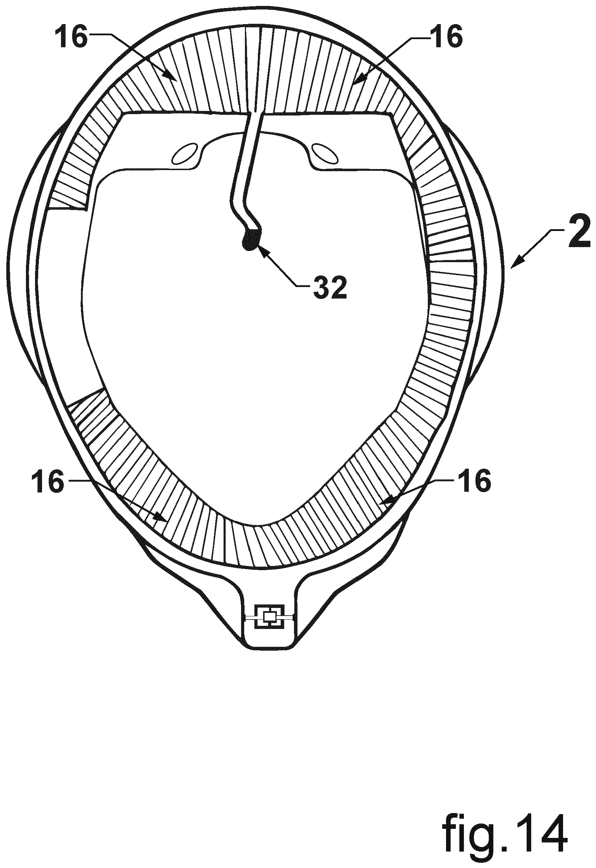

In **figure 14****,** the top view of the wreath (2) is illustrated. The only difference in comparison to figure 3 is the type of the interior soft inlay (16), which is an adjustable bag containing air, or air and a soft material, or liquid, or air and liquid. Air or liquid intake or outtake is performed through the valve (32) to such an amount in order to create a comfortable cushion covering the skull relief individually.

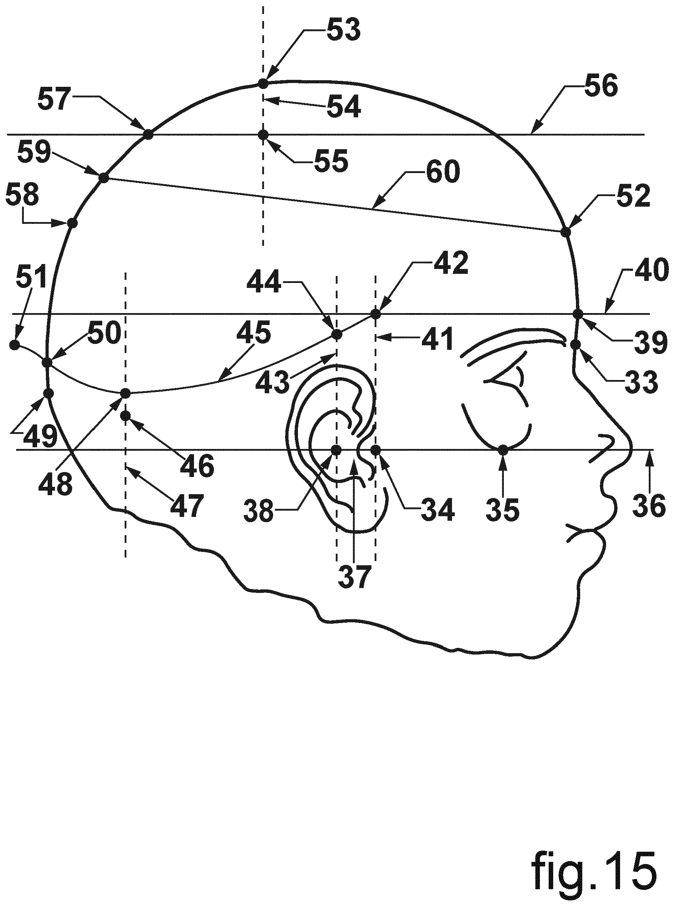

In **figure 15****,** the references (points, lines and linear segments) used in the digital design of the caudal and cranial boundaries of the wreath (2) are presented.
   The following craniometric points are defined on the editable surface of the patient's head and all of them are projected on the midsagittal plane: the Glabella (33); the Tragion (34); the Orbitale (35); the point (39) on the frontal bone contour located ten mm above Glabella; the Sub-Inion right (46); the Inion (49); the point (52) located on the frontal bone contour and 28 mm above the point 39; the apex (53) of the patient's head; the apex (58) of the curve located on the dorsal contour of the patient's head between the points 49 and 57; the point (57) located on the dorsal head contour, where the line 56 intersects this contour; the line (56) is parallel to Frankfort horizontal plane (36) and crosses the point 55; the point (55) located twenty mm below the craniometric point 53 on the line 54; the line (54) crosses the point 53 and is perpendicular to the Frankfort horizontal plane (36); the point (59) on the dorsal contour of the head located thirty mm above the point 58.
   The following additional points are presented: the point (38), which is the middle of the concha (37) of the external ear on the Frankfort horizontal plane (36); the point (42), which is the point where the line 41 intersects the line 40 and it is the first control point of the spline curve (45); the line (41) perpendicular to the line 36 through the craniometric point 34; the line (40) crosses the craniometric point 39 and is parallel to 36; the second control point (44) of the spline curve (45) is located 2 mm more caudally to the point 42 and on the line 43; the line (43) is perpendicular to the line 36 through the point 38; the third control point (48) of the spline curve (45) is located on the line 47 and five mm above the point 46; the line (47) is perpendicular to the line 36 crossing the craniometric point 46; the fourth control point (50) of the spline curve (45) is located five mm above the point 49 and the last free point (51) of the spline curve (45) to relax tensions on the curve.
   The spline curve (45) consisting of these four control points and one free point at its end is defined on the midsagittal plane. Its projected surface, which is perpendicular to the midsagittal plane, comes out to define the dorsal part of the caudal boundaries of the wreath (2) on the head surface of the patient. As with the spline curve (45), all the points, lines and linear segments are defined on the midsagittal plane. The perpendicular projection to the midsagittal plane of the linear segment 52-59 on the head surface represents the digital cranial boundaries of the wreath (2). The linear segment 39-42 is parallel to the Frankfort horizontal plane (36). Finally, the digital contour defined through the points, 39, 52, 59, 50, 48, 44, 42, 39, represents the projected profile contour of the digital designed wreath (2).

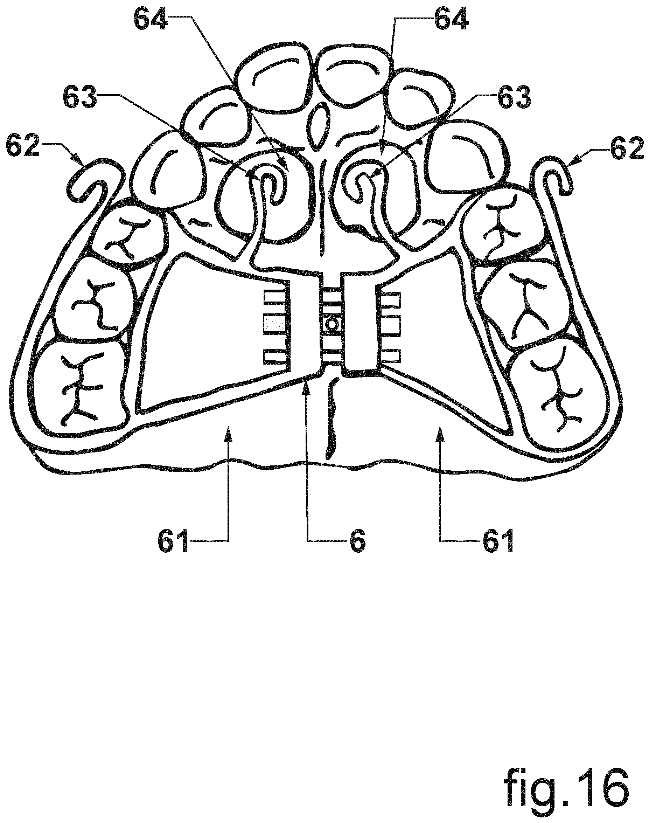

In **figure 16****,** the right and left maxillary halves (61) of a patient with a conventional mechanism of rapid maxillary expansion (6), banded on four maxillary teeth, are presented. On the lateral sides of the RME mechanism, the two welded metallic bars with their circular bends (62) at their frontal ends responsible for the attachment of the traction means (5), are also illustrated. In this traditional RME mechanism, two components on its frontal area are added. The connecting wires (63), between RME mechanism and acrylic pads (64), which comprise the frames for the acrylic pads (64), are illustrated. The acrylic pads (64) are located in very close contact with the palatal mucosa in the frontal region of the maxilla, right and left.

### DEVICE OPERATION

Initially, "rapid maxillary expansion" using the traditional method is performed by any intraoral device [1-4, 8]. As an example, a modified "Hyrax" device (6, fig.16) banded to the maxillary posterior teeth by glass-ionomer cement is mentioned. In the buccal aspects of the bands, metallic bars, which have a circular bend (62) at their frontal ends, usually in the canine area, in order to be intraorally attached to the elastic bands, are welded. On the first permanent maxillary molars and on the second deciduous maxillary molars, when they are in the mouth, fixed bite-planes (posterior bite turbos) of a visible light-cure material, are placed. They are about 3 mm in height and they are used in order to impede maxillary molar extrusion, which is followed by downward and backward rotation of the mandible, by the opening of the midpalatal suture by the use of the "Hyrax" device. The bite planes are controlled on each visit to check for occlusal imprints of the mandibular teeth, which would obstruct forward maxillary movement and are repaired accordingly.

The extraoral orthopedic device is applied immediately after the disarticulation of the circumaxillary sutures aiming at an adequate maxillary protraction. After the end of the maxillary expansion and directly before the maxillary traction, two individualized acrylic pads (64), which had been manufactured on the palatal mucosa of the two maxillary halves in the front region of the maxilla, are placed at very close contact with the palatal mucosa and are connected with the two halves of the expansion screw by means of two additional wires (63), which had been supplemented on the conventional RME mechanism before its insertion. These two acrylic components (64), as additional anchorage, contribute to a "pure" maxillary traction preventing the maxillary support-teeth from being mesially moved because of the protractive forces, which reduces the orthopedic outcome and might result in an anterior crowding.

**First,** the skeletal support wreath is adjusted to the person's skull with the help of the interior soft inlay (16, fig.3, 14) in the form of soft pads or an adjustable bag containing air, or air and soft material, or liquid, or liquid and air, producing maximum comfort and fit. **Second,** the connecting (3) girder of the device (1), after being inserted into the cuboidal slot (12, fig.3) of the wreath (2), is initially adjusted in height telescopically in relation to the patient's mouth slit and fixed by means of fixing screws (15, fig.2, 3). Then, the mouth girder (4, fig.1, 2, 6), after being inserted into the connecting girder (3) through its cuboidal slot (25), is also telescopically adjusted in height in order for the person's jaw to receive an optimal force direction for its traction. The immobilization in the connecting girder is achieved by means of the fixing screws (21, fig.1, 6). **Third,** the wreath with the attached two girders is removed from the patient's head and the two elastic fixation straps (7) are connected with it through its elliptical slits (17) in their fixation areas (26). After that, the caudal ends of the straps coming through their buckles create a strap-loop in an anterior-posterior direction on each side of the wreath (fig.2, 4, 7). **Fourth,** traction means, such as elastic bands (5, fig.2), are applied in order to protract the maxilla. Initially, the traction means are attached intraorally on the lateral hooks (62, fig.16) of the intraoral device (6, fig.2 and 16). Then, the arms of the patient come through the inner side of the aforementioned strap-loops and the cranial support wreath (2, fig.1-4, 7) is primarily adapted (connecting girder in the middle of the face) and fixed to the patient's neurocranium (9, fig.1, 2, 4, 7) through its perfect shape and its interior soft inlay (16, fig.3, 14). **Fifth,** the two elastic fixation straps (7) are initially activated pulling the wreath downwards from its dorsal side. Then, the traction means (5) are attached extraorally to the two elongated elements with the mounting means, such as mounting screws (20, fig.2), of the mouth girder (4). Their pulling forces and their tractionangle are measured and are finally adjusted in relation to the patient's needs. If the protracting forces bring the connecting girder close to the patient's chin moving upwards and forwards the dorsal side of the wreath, the elastic fixation straps are additionally activated in such a way until an equilibrium occurs between the forces developed by the operation of the device (1) in its frontal and dorsal area, which is essential for its proper function. This last activation of the straps (7) is of vital importance because the risk of dislodgment during use of the device is virtually eliminated and additionally the distance between the mouth girder (4) and the patient's mouth slit remains unchanged (stable traction forces).

The extraoral attachment of the traction means is achieved ergonomically and symmetrically on the right and left side of the patient's head, also in respect to the patient's rima oris width, avoiding a trauma of the oral commissures, thanks to the various attachment positions (23, fig.6) on the mouth girder (4). The mandible could also be directly protracted using an additional mouth girder (4), placed upside down (traction means 10 - intraoral mechanism fixed in the mandible 11, fig.7).

Summarizing, it is emphasized that this is an extraoral orthopedic device which can greatly help in the therapeutic modification of the maxillary and mandibular growth in skeletal Class III and Class II patients when it is especially used in cooperative, growing young individuals. Its main advantages are:
**1.** Because of its skeletal anchorage, primarily to the neurocranium and partly around the shoulders and through the axillae of each patient during maxillary protraction, there is no pressure on the mandible, thus avoiding any adverse effect on the temporomandibular joints arising from increased pressure during its use. Consequently, an enlargement of the oral cavity occurs with all the benefits that this implies.
**2.** Easy use of the device, not only in the therapy of skeletal Class III patients, but for the aetiologic therapy of skeletal Class II patients, even in the cases of Angle Class I or Class II malocclusions with retrognathic mandible but also retrognathic maxilla within the face, which, until now, is not the case by using extraoral or intraoral devices. Using this device, the mandible can also be protracted directly or indirectly. Particular mention of the use of this device is also made for operated cleft lip and palate patients, whose maxilla is often retrognathic due to the scars created by the operations on the upper lip and maxilla. These scars impede the physiologic growth of the maxilla, but the mandible usually has a physiologic position in the patient's skull.
**3.** Optimum symmetry in device settings when our device is used on the patient's skull in a versatile and safe manner. The applying traction means can be attached in the direction desired by the orthodontist both vertically and transversely, due to the ergonomic and practical design.

Initially, the fully individualized wreath, based on the head scan, is manufactured. Additionally, the head perimeter of the patient is measured at the beginning of treatment. The thickness of the initial interior soft inlay, which partly covers the inner surface of the wreath, could be large enough in order that it can be replaced with a thinner interior soft inlay, in the case that within the therapy-period of time minor growth of the skull occurs. It becomes even easier in the case where the interior soft inlay is an adjustable bag containing air, or air and soft material, or liquid, or liquid and air. In this case, the thickness of the interior soft inlay can be reduced by removing air from the adjustable bag. On each visit, it is recommended that the head perimeter of the developing individual be measured, in order to regulate physiological enlargement of the human skull for each patient. If the skull growth, within this period of time, is significant, the wreath has to be replaced with a new, wider one adapted to the new skull dimensions.

Lastly, parts of this device could be manufactured with other materials. Indicatively, the carbon fiber material for the construction of the cranial support wreath is mentioned, which is extremely lightweight and durable. The wreath could also be manufactured from Nylon (PA11 or PA12). Other materials, such as PLA with similar properties to those of thermoplastic (ABS) could be used, but with a more ecological footprint (biodegradable and compostable). Both girders could consist of an aluminum-alloy or of a stainless steel-alloy or a combination of them, materials that can be reused after sterilization. Velcro type fasteners, clamping regulators between the skeletal support wreath sections could also be integrated. Different thicknesses in the interior soft inlay, when it is offered only as a soft material pad and not as an adjustable air- and/or liquid-bag, depending on the hair volume of each individual or to adjust to the physiologic growth of the human skull, could be an additional service to each person.

The elastic fixation straps used for the proper fixation of the wreath and against its dislodgment would be able to pass, apart from through the axillae (fig.2, 4, 7) and the groins, (fig.12), as already described, under the bottom of the feet (sole, plantar aspect). These are the three natural areas of the human body, that could offer the required stability for the wreath. The first mentioned body area (axilla) is closer to the wreath, more comfortable for the patient and more controlled.

### METHOD OF DESIGN AND MANUFACTURING OF THE WREATH USING 3D DIGITAL DATA ACQUISITION RECEIVED AFTER SCANNING A PERSON'S HEAD

The use of non-invasive methods, which would not harm the person's health, for the manufacture of a 3D printed customized facemask has previously been used [30]. In this way, the use of the relatively high dose of radiation involved in CBCTs [31-36] is avoided.

Cutting-edge technology at the current level of science, such as with a hybrid LED and Infrared Light Source Handheld Color 3D scanner is used in the workflow of an accurate creation of the skeletal support wreath involved in the herein-described device. This is achieved either by digitizing the anatomy of the person's head, or by digitizing the geometry of the "wreath" created with impression material, as described below and with the help of reverse engineering.

### Preparation before scanning (fig.8, 9)

**a.** An elastic cover, such as an elastic wig cap or an elastic swimming cap, is placed on the person's head. If the person has excessively thick hair, an elastic cap with a perforation on its top could be used, so that the greater volume of hair can be collected outside the elastic cap and kept away from the scanning areas where the skeletal support wreath is placed.
**b.** Subsequently and before scanning, it may be useful through palpation to determine concrete anatomical points on the person's skull and to place self-adhesive dot-scannable stickers on the following nine anatomical (craniometric) points: the Glabella, the left and right Orbitale, the left and right Tragion, the Labrale superior, the Inion (the outermost craniometric point of the external occipital protuberance) and the left and right outermost points of the prominences between the superior and inferior nuchal lines on the caudo-lateral areas of the occipital bone [the Sub-Inion left (SInL) and Sub-Inion right (SInR)].
**c.** The person's head is placed in its natural head position (NHP), which implies that the head is in an upright posture, the eyes focused on a point in the distance at eye level, which indicates that the visual axis is horizontal.

### Digital workflow (fig.13)

Next, the scanning of the person's head follows. After that, a 3D point cloud is obtained, which is generated into a 3D mesh model of the head. Then, this is generated into a 3D digital imprint of the head in the form of a 3D file, such as an stl file, including locating on a reference coordinate system the coordinates of a plurality of anatomical points of the subject involving at least the following points: the Glabella, the left and right Orbitale, the left and right Tragion, the Labrale superior, the Inion and the outermost points of the prominences between the superior and inferior nuchal lines on the caudo-lateral areas of the occipital bone, i.e. the Sub-Inion left and Sub-Inion right.

After that, the 3D file is imported to a CAD software program and the whole skull transformed into an editable area followed by the definition of the aforementioned anatomical points. If the computer capacity is not adequate, the small areas around and including the aforementioned anatomical points are transformed into editable areas and defined. Then, the definition of a midsagittal plane including the Glabella, the Labrale superior and the Inion and a Frankfort horizontal plane including at least one of the following pairs of points: the left Orbitale with the left Tragion or the right Orbitale with the right Tragion, and which is normal to the midsagittal plane, is carried out. Following this, the generation of a 3D reconstruction editable surface of the wreath's area on the head is performed, which is unnecessary in the case that the whole skull has already been transformed into an editable surface. Subsequently, the creation of the caudal and cranial boundaries of the wreath surface using as references the midsagittal and Frankfort horizontal planes, as well as at least the points, lines and linear segments (33 - 60) mentioned in the Figure 15, is carried out. Next, a general offsetting in an upward scaling to accommodate the hair volume of the person and the thickness of the interior soft inlay, in either form, is completed.

Then, a solid model of the wreath is generated.

The designing and positioning of the slot (12) for the universal connecting girder (3) in the frontal area of the wreath, the center of which is located on the midsagittal plane, follows. The procedure continues with the designing and positioning of the reinforcement webs (18) in the frontal and lateral areas of the wreath.

After that, a negative offsetting in the frontal and dorsal area of the wreath on the areas which are going to accommodate the interior soft inlay, is performed in the case where the interior soft inlay is offered as soft material pads, but not for an adjustable bag containing air, air and soft material, liquid or air and liquid.

In the end, the designing and positioning of the elliptical slits (17) in the dorsal area of the wreath is carried out. The minor axes of these two elliptical slits pass through the line sections AxL-SInL and AxR-SInR (fig.10) in the case where the two elastic fixation straps (7) pass through the axillae and around the shoulders or through the line sections GrL-SInL and GrR-SInR (fig.11) in the case where the two elastic fixation straps (7) pass through the groins and around the thighs. The major axes of the two elliptical slits are perpendicular to their minor axes, as occurs in any ellipse. The lengths of the minor and the major axes of the elliptical slits are determined by the thickness and the width of the elastic fixation straps (7), correspondingly. The centers of the left and right elliptical slits are determined circa 25 mm above the SInL and SInR on the line sections AxL-SInL and AxR-SInR or GrL-SInL and GrR-SinR correspondingly, as well as the vertices (the points where the major axes cut the ellipses) and the lower co-vertices (the points where the minor axes cut the ellipses caudally) are at least 1 cm apart from the caudal boundaries of the wreath. The extensions of the line sections AxL-SInL and AxR-SInR create the angles L and R (fig.10) with the line crosses the anatomical points In and Ap. These two angles are used in the digital design of the two elliptical slits (17), when the applied elastic fixation straps (7) pass through the axillae (fig.2, 4, 7). The extensions of the line sections GrL-SInL and GrR-SInR create the angles L' and R' (fig.11) with the line crosses the anatomical points In and Ap. These two angles are used in the digital design of the two elliptical slits (17), when the applied elastic fixation straps (7) pass through the groins (fig.12).

The next stage involves the generation of a 3D solid model file of the digitally designed wreath, such as an stl file (CAD workflow file).

After that, the 3D solid model file of the wreath is placed into a 3D printing slicer software, which transforms this digital model into printing instructions (G code file).

Finally, the wreath is manufactured by means of a 3D printer (CAM procedure).

The above-mentioned digital procedure can be followed using **two modes of digital 3D data acquisition:**
First, the wreath is roughly created directly on the skull within a traced contour by means of an impression material, such as polysiloxane (addition silicone type) or a thermoplastic material, such as those used in the manufacture of masks to immobilize the person's head, in particular during radiotherapy (radiotherapy immobilization devices). After the polymerization of the impression material, the inner surface of the "wreath", which corresponds to the anatomy of the outer surface of the neurocranium, is scanned. In this way, the geometry of the neurocranium, created by means of the impression material, using a reverse engineering technique, is digitized.
Secondly, the best method, ergonomically and environmentally, for the CAD-CAM creation of the wreath is **direct scanning of the person's head.** Then, point processing and geometric model development is carried out using computer soft-ware and finally, it is printed using a 3D printer.

A computer program could be created comprising instructions which, when the program is executed by a computer, cause the computer to carry out certain steps of the above-mentioned method.

Its effectiveness in terms of the skeletal support of the inventive extraoral orthopedic device and the comfort of the person during its application through this design, is superior, because it correlates with exceptional precision to the anatomy of the head of each person. It is no longer **an ellipsoidal wreath,** but **a wreath of individualized cranial form,** which corresponds perfectly to the anatomy of the person's head. Measurements **by mechanical means,** in our case, measurement with the 3D structured-light scanner, are not associated with any **subjective errors.** The prerequisite is of course that the digital impression instructions (data acquisition instructions), mentioned above, have been strictly followed, as well as the manufacturer's instructions, such as the correct setting (calibration) of the 3D scanner (scanning distance, room brightness, etc.). The only disadvantage of this technique is the need to process large information files, but this is easily overcome by using computers capable of processing such large 3D files.

### OTHER APPLICATIONS

It can also be mentioned that this technique of digitally imaging human skulls with a 3D scanning camera using structured light and with the aid of the elastic cover, as described above, can be used to create protective helmets, which can be used in the context of various activities (sports, workplaces, etc.) where a head cover is deemed necessary for protection and safety reasons. A second application of this method could be to create individualized symmetrical helmets worn especially during the first year of age of individuals presenting asymmetries in their skull morphology as symmetry guidance.

### REFERENCES

1. Haas AJ. Rapid expansion of the maxillary dental arch and nasal cavity by opening the midpalatal suture. Angle Orthod. 1961;31(2):73-90.
2. Biederman W. A hygienic appliance for rapid expansion. J Pract Orthod. 1968;2(2):67-70.
3. Haas AJ. Palatal expansion: Just the beginning of dentofacial orthopedics. Am J Orthod. 1970;57(3):219-55.
4. McNamara JA. An Orthopedic Approach to the Treatment of Class III Malocclusion in Young Patients. J Clin Orthod. 1987;21(9):598-608.
5. Liou EJ, Tsai WC. A new protocol for maxillary protraction in cleft patients: repetitive weekly protocol of alternate rapid maxillary expansions and constrictions. Cleft Palate-Craniofacial Journal, 2005;42:121-27.
6. Canturk BH, Celikoglu M. Comparison of the effects of face mask treatment started simultaneously and after the completion of the alternate rapid maxillary expansion and constriction procedure. Angle Orthod. 2015;85(2):284-91.
7. Liu W, Zhou Y, Wang X, Liu D, Zhou S. Effect of maxillary protraction with alternating rapid palatal expansion and constriction vs expansion alone in maxillary retrusive patients: A single-center randomized controlled trial. Am J Orthod Dentofacial Orthop. 2015;148(4):641-51.
8. Wilmes B, Nienkemper M, Ludwig B, Kau CH, Drescher D. Early Class III Treatment with a Hybrid-Mentoplate Combination. J Clin. Orthod. 2011;45(1):1-7.
9. Delaire J. Confection du masque orthopedique. Rev Stomatol. 1971;72(5):579-84.
10. Delaire VJ, Verdon P, Floor J. Ziele und Ergebnisse extraoraler Züge in posteroanteriorer Richtung in Anwendung einer orthopädischen Maske bei der Behandlung von Fällen der Klasse III. Fortschr Kiefer Orthop 1976;37:246-262.
11. Petit H. Adaptations following accelerated facial mask therapy in clinical alteration of the growth face. In: McNamara JA Jr, Ribbens KA, Howe RP (eds). Clinical Alteration of the Growing Face, monograph 14, Craniofacial Growth Series. Ann Arbor, MI: University of Michigan, 1983.
12. Turley PK. Orthopedic correction of Class III malocclusion with palatal expansion and custom protraction headgear. J Clin Orthod. 1988;22(5):314-325.
13. https://www.orthazone.com/Face-Mask-ReversePull-Headgear-T%C3%BCbinger-Model-dtu-109
14. https://www.google.com/search?client=firefox-b-d&q=sky+hook+head+gear
15. Hegmann M, Rüther AK.The Grummons face mask as an early treatment modality within a class III therapy concept. J Orofacial Orthop. 2003;64(6):450-6.
16. https://www.leone.it/english/services/download/Cat_Orthodontic_Eng.pdf
17. Alcan T, Keles A, Erverdi N. The effects of a modified protraction headgear on maxilla. Am J Orthod Dentofacial Orthop. 2000;117:27-38.
18. De Clerck HJ, Cornelis MA, Cevidanes LH, Heymann GC, Tulloch CJ. Orthopedic traction of the maxilla with miniplates: a new perspective for treatment of midface deficiency. J Oral Maxillofacial Surg. 2009;67(10):2123-9.
19. Yatabe M, Garib D, Faco R, de Clerck H, Souki B, Janson G, Nguyen T, Cevidanes L, Ruellas A. Mandibular and glenoid fossa changes after bone-anchored maxillary protraction therapy in patients with UCLP: A 3-D preliminary assessment. Angle Orthod. 2017 May;87(3):423-431.
20. Proffit, William, R. et al. Treatment of Skeletal Transverse and Class III Problems (chapter 13). Contemporary Orthodontics (6th Edition). Elsevier - OHCE, 2018.
21. Cha BK, Choi DS, Ngan P, Jost-Brinkmann PG, Kim SM, Jang Is. Maxillary protraction with miniplates providing skeletal anchorage in a growing Class III patient. Am J Orthod Dentofacial Orthop. 2011;139:99-112.
22. Pourcho WS. Orthodontic appliance. U.S. Patent number: 5158451, Oct 27, 1992.
23. Ngiam loachim. Mandibular advancement. U.S. Patent number: 0040301, Feb.16,2012.
24. DEL GOBBO WANDA. ORTHOPEDIC DEVICE FOR THE PROTRACTION OF THE MAXILLARY ARC. CPC: A61C7/06, IPC: A61C7/06, Publication info: WO2017089971 (A1) 2017-06-01.
25. KAHN SANDRA VIVIAN, FLORES CERCEDA MABEL, KAHN LEVENTHAL ILAN. MAXILLARY PROTRACTION DEVICE. CPC: A61B2560/0475, A61B5/0008, A61B5/01, IPC: A61B5/00, A61B5/01, A61C7/06, Publication info: US2018028282 (A1), 2018-02-01; US10166089 (B2) 2019-01-01.
26. KOUTZOGLOU STYLIANOS, KOUTZOGLOU ELENI. EXTRAORAL ORTHOPEDIC DEVICE FOR THE DIRECT PROTRACTION OF THE MAXILLA AND THE INDIRECT PROTRACTION OF THE MANDIBLE. IPC: A61C7/06; A61C7/36; A61F5/56; CPC: A61C7/06 (EP, GR); A61C7/36 (GR); A61F5/05891 (EP); A61F5/56 (EP, GR); A61C7/36 (EP); Publication info: WO2021090035A1 Doi:https://worldwide.espacenet.com/patent/search/family/071120204/pubiication/WO20 21090035A1?q=WO%202021%2F090035
27. Lorente C, Alfaro FH, Vela MP, Lorente P, Lorente T. Surgical-Orthodontic approach for facial rejuvenation based on a reverse facelift. Progress in Orthodontics (2019) 20:34 https://doi.org/10.1186/s40510-019-0287-8
28. Schwarz A. M.: Die Röntgenostatik. Urban & Schwarzenberg, Wien-Innsbruck 1958.
29. Yatabe M., Garib D.G., André de Souza Faco R, de Clerck H., Janson G, Nguyen T., Lucia Helena Soares Cevidanes L.H.S., Ruellas A.C. Bone-anchored maxillary protraction therapy in patients with unilateral complete cleft lip and palate: 3-dimensional assessment of maxillary effects. Am J Orthod Dentofacial Orthop. 2017 Sep;152(3):327-335 doi: 10.1016/j.ajodo.2016.12.024
30. Franchi L, Vichi A, Marti P, Lampus F, Guercio S, Recupero A, Giuntini V, Goracci C. 3D Printed Customized Facemask for Maxillary Protraction in the Early Treatment of a Class III Malocclusion: Proof-of-Concept Clinical Case. Materials 2022, 15(11), 3747; https://doi.org/10.3390/ma15113747
31. Hans MG, Palomo JM, Valiathan M. History of imaging in orthodontics from Broadbent to cone-beam computed tomography. Am J Orthod Dentofacial Orthop. 2015;148:914-21. DOI:https://doi.org/10.1016/j.ajodo.2015.09.007
32. Velasco Regina Casian Ruiz, Liang Hui Cone-Beam Computed Tomography (CBCT) Applications in Dentistry. DOI: https://www.dentalcare.com/en-us/professional-education/ce-courses/ce531/overview
33. Yeh, JK., Chen, CH. Estimated radiation risk of cancer from dental cone-beam computed tomography imaging in orthodontics patients. BMC Oral Health 18, 131 (2018). DOI:https://doi.org/10.1186/s12903-018-0592-5
34. Dental Cone-beam Computed Tomography https://www.fda.gov/radiation-emitting-products/medical-x-ray-imaging/dental-cone-beam-computed-tomography#benefitsrisks
35. Safety and Efficacy of a New and Emerging Dental X-ray Modality. https://cordis.europa.eu/project/id/212246
36. Pediatric X-ray imaging https://www.fda.gov/radiation-emitting-products/medical-imaging/pediatric-x-ray-imaging

### TABLE OF REFERENCE SIGNS OF DRAWINGS

| | |
|---|---|
| **1:** | The extraoral orthopedic device for the protraction of the human jaws |
| **2:** | The skeletal support wreath |
| **3:** | The connecting girder placed in the middle of the wreath on its frontal area |
| **4:** | The mouth girder |
| **5:** | The traction means, such as elastic bands, attached extraorally to the elongated elements with their mounting means, such as mounting screws (20), and intraorally to the mechanism of "rapid maxillary expansion" (6) in the maxilla. |
| **6:** | The intraoral mechanism fixed in the maxilla |
| **7:** | The elastic fixation straps of the skeletal support wreath |
| **8:** | The axilla |
| **9:** | The person's neurocranium |
| **10:** | The traction means, such as elastic bands, attached extraorally to the elongated elements with their mounting means, such as mounting screws (20), and intraorally to a mechanism (11) fixed in the mandible. |
| **11:** | The intraoral mechanism fixed in the mandible |
| **12:** | The cuboidal slot in the frontal area of the wreath (2) responsible for the passage of the connecting girder (3). |
| **13:** | The metallic parts which create the cuboidal slot (12) in the skeletal support wreath. |
| **14:** | The cylindrical hole in the wreath guiding the fixing screw (15) |
| **15:** | The fixing screw for the immobilization of the connecting girder (3) |
| **16:** | The interior soft inlay of the skeletal support wreath (2) in the form of a soft material pad or an adjustable bag containing air, or air and a soft material, or liquid, or air and liquid |
| **17:** | The elliptical slits in the dorsal area of the wreath for the passage of the cranial end of the elastic fixation straps (7) |
| **18:** | The reinforcement webs, or anti-bend supports, to prevent bending of the wreath (2) in its frontal and lateral areas during use of the device (1) |
| **19:** | The markings on the cranial part (28) of the connecting girder (3) responsible for an easy and accurate replacement and adjustment in its height in the wreath (2) and the markings on the caudal part (30) of the connecting girder (3) responsible for an easy and accurate replacement and adjustment in the height of the mouth girder (4) along the connecting girder (3). |
| **20:** | The elongated elements with a mounting means, such as mounting screws on the mouth girder (4), responsible for the attachment of the traction means (5 or 10), such as elastic bands. |
| **21:** | The fixing screws for the immobilization of the mouth girder (4) |
| **22:** | The threaded holes for the anterior and posterior fixing screws (21) responsible for the immobilization of the mouth girder (4). |
| **23:** | The threaded holes responsible for the accommodation of the elongated elements with mounting means (20). |
| **24:** | The separate aluminum-alloy part incorporated into the mouth girder (4) through the fixing screws (21) responsible for the immobilization of this girder. |
| **25:** | The cuboidal slot for the passage of the connecting girder (3) |
| **26:** | The attachment of the cranial end of the elastic fixation straps (7) |
| **27:** | The buckle responsible for the adjustment of the elastic fixation straps in height and to their pulling-power. |
| **28:** | The cranial part of the connecting girder (3) |
| **29:** | The middle part of the connecting girder (3) |
| **30:** | The caudal part of the connecting girder (3) |
| **31:** | The groin |
| **32:** | The valve of the adjustable bag (16) for the individualized air or liquid intake or outtake |
| **33:** | The craniometric point Glabella (G) |
| **34:** | The craniometric point Tragion (Trg) |
| **35:** | The craniometric point Orbitale (Or) |
| **36:** | The Frankfort horizontal plane |
| **37:** | The concha of external ear |
| **38:** | The middle of the concha on the Frankfort horizontal plane |
| **39:** | The craniometric point on the frontal bone contour located ten mm above Glabella |
| **40:** | The line crossing the craniometric point 39 and parallel to 36 |
| **41:** | Perpendicular to the Frankfort horizontal plane through the craniometric point 34 |
| **42:** | The point where line 41 intersects line 40 and is the first control point of the spline curve 45. |
| **43:** | Perpendicular to the Frankfort horizontal plane through the point 38 |
| **44:** | The second control point of the spline curve 45, located two mm more caudally to the point 42 and on the line 43 |
| **45:** | The spline curve consisting of four control points and one free point at its end |
| **46:** | The craniometric point Sub-inion right (SInR) |
| **47:** | Perpendicular to Frankfort horizontal plane crossing the craniometric point 46 |
| **48:** | The third control point of the spline curve 45, which is located on the perpendicular 47 and five mm above the craniometric point 46. |
| **49:** | The craniometric point Inion (In) |
| **50:** | The fourth control point of the spline curve 45, which is located five mm above the craniometric point 49 on the dorsal contour of the head. |
| **51:** | The free point of the spline curve at its end |
| **52:** | The craniometric point located on the frontal bone contour twenty-eight mm above the craniometric point 39. |
| **53:** | The apex of the patient's head |
| **54:** | The perpendicular to Frankfort horizontal plane crossing the craniometric point 53 |
| **55:** | The point on the line 54 located twenty mm below the craniometric point 53. |
| **56:** | The line parallel to Frankfort horizontal plane crossing the point 55 |
| **57:** | The craniometric point, which is located on the dorsal contour of the patient's head, where the line 56 intersects the head contour. |
| **58:** | The apex of the curve of the patient's head between the craniometric points 49 and 57 |
| **59:** | The craniometric point on the dorsal contour of the head located thirty mm above the craniometric point 58 |
| **60:** | The linear segment 52-59 representing the digital cranial boundaries of the wreath (2) |
| **61:** | The right and left halves of the maxilla |
| **62:** | The right and left circular bends on the welded metallic bars responsible for the attachment of the traction means (5). |
| **63:** | The connecting wires between RME and acrylic pads (64) |
| **64:** | The right and left acrylic pads on the frontal palatal mucosa right and left sections correspondingly |

## Claims

1. A computer-implemented method of designing an individualized skeletal support wreath (2), the computer-implemented method comprising:
. obtaining a 3D point cloud of a head;
. generating a 3D mesh model of the head;
. generating a 3D digital imprint of the head in the form of a 3D file, such as an stl file, including:
. locating on a reference coordinate system the coordinates of a plurality of anatomical points of the subject including at least the following points: the Glabella, the left and right Orbitale, the left and right Tragion, the Labrale superior, the Inion (In), and the outermost points of the prominences between the superior and inferior nuchal lines on the caudo-lateral areas of the occipital bone, i.e. the Sub-Inion Left (SInL) and Sub-Inion Right (SInR);
. importing of the 3D file to a CAD software program;
. transforming into editable areas the whole head or at least the small areas around and containing the aforementioned anatomical points and the defining of these points;
. defining a midsagittal plane including the Glabella, the Labrale superior and the Inion and a Frankfort horizontal plane including at least one of the following pairs of points:
the left Orbitale with the left Tragion or the right Orbitale with the right Tragion, and being normal to the midsagittal plane;
. generating a 3D reconstruction editable surface of the individualized skeletal support wreath's (2) area on the head;
. creating the caudal and cranial boundaries of the individualized skeletal support wreath (2) surface using as references the midsagittal and Frankfort horizontal planes and at least the points, lines and linear segments (33 - 60);
whereby said points, lines and linear segments (33 - 60) include at least one of the following:
the craniometric point Glabella (G, 33)
the craniometric point Tragion (Trg, 34)
the craniometric point Orbitale (Or, 35)
the Frankfort horizontal plane (36)
the concha (37) of external ear
the middle (38) of the concha (37) on the Frankfort horizontal plane (36)
the craniometric point (39) on the frontal bone contour located ten mm above Glabella (33)
the line (40) crossing the craniometric point (39) and parallel to the Frankfort horizontal plane (36)
the perpendicular (41) to the Frankfort horizontal plane (36) through the craniometric point (34)
the point (42) where the perpendicular line (41) intersects the line (40) and is the first control point of the spline curve (45)
the perpendicular (43) to the Frankfort horizontal plane (36) through the point (38)
the second control point (44) of the spline curve (45), located two mm more caudally to the point (42) and on the perpendicular (43)
the spline curve (45) consisting of four control points and one free point at its end the craniometric point Sub-Inion Right (SInR, 46)
the perpendicular (47) to Frankfort horizontal plane (36) crossing the craniometric point (46)
the third control point (48) of the spline curve (45), which is located on the perpendicular (47) and five mm above the craniometric point (46) the craniometric point Inion (In, 49)
the fourth control point (50) of the spline curve (45), which is located five mm above the craniometric point (49) on the dorsal contour of the head
the free point (51) of the spline curve (45) at its end
the craniometric point (52) located on the frontal bone contour twenty-eight mm above the craniometric point (39)
the apex of the patient's head (53)
the perpendicular (54) to Frankfort horizontal plane (36) crossing the apex of the patient's head (53)
the point (55) on the perpendicular (54) located twenty mm below the apex of the patient's head (53)
the line (56) parallel to Frankfort horizontal plane (36) crossing the point (55) the craniometric point (57) located on the dorsal contour of the patient's head, where the line (56) intersects the head contour
the apex of the curve of the patient's head (58) between the craniometric points (49) and (57)
the craniometric point (59) on the dorsal contour of the head located thirty mm above the craniometric point (58)
the linear segment (60) between the craniometric points (52) and (59) representing the digital cranial boundaries of the individualized skeletal support wreath (2),
. offsetting in an upward scaling to accommodate the hair volume of the patient and the thickness of an interior soft inlay (16) of the individualized skeletal support wreath (2);
. generating a solid model of the individualized skeletal support wreath (2);
. designing and positioning of a slot (12) of the individualized skeletal support wreath (2) for a connecting girder (3) in the frontal area of the individualized skeletal support wreath (2), the center of which is located on the midsagittal plane;
. designing and positioning of reinforcement webs (18) in the frontal and lateral areas of the individualized skeletal support wreath (2);
. designing and positioning of elliptical slits (17) for the passage of a pair of elastic straps (7) in the dorsal area of the individualized skeletal support wreath (2), determined through angles L and R when using axilla (8) support and determined through angles L' and R' when using groin (31) support,
wherein the extensions of a line section passing through the left axillae (AxL) and the craniometric point Sub-Inion Left (SlnL) and a line section passing through the right axillae (AxR) and the craniometric point Sub-Inion Right (SInR) create the angles L and R with a line crossing the craniometric point Inion (In, 49) and the apex of the patient's head (Ap, 53) and the extensions of a line section passing through the left groins (GrL) and the craniometric point Sub-Inion Left (SlnL) and a line section passing through the right groins (GrR) and the craniometric point Sub-Inion Right (SInR) create the angles L' and R' with a line crossing the craniometric point Inion (In, 49) and the apex of the patient's head (Ap, 53);
. generating a 3D solid model file of the digitally designed individualized skeletal support wreath (2), such as an stl file.

2. The computer-implemented method of designing the individualized skeletal support wreath (2) according to claim 1, in which a 3D digital imprint of a head is obtained by use of a hybrid LED and Infrared Light Source Handheld Color 3D scanner.

3. The computer-implemented method of designing the individualized skeletal support wreath (2) according to claims 1 or 2, whereby the individualized skeletal support wreath (2) forms part of a protective helmet or part of a helmet for the guidance of symmetrical skull growth.

4. The computer-implemented method of designing the individualized skeletal support wreath (2) of claims 1-3, further including the step of
. placing the 3D solid model file of the individualized skeletal support wreath (2) into a 3D printing slicer software, transforming this digital model into printing instructions; and
. manufacturing of the individualized skeletal support wreath (2) by means of a 3D printer.

5. A computer program comprising instructions which, when the program is executed by a computer, cause the computer to carry out the following steps of the method of claim 1,
. defining a midsagittal plane including the Glabella, the Labrale superior and the Inion and a Frankfort horizontal plane including at least one of the following pairs of points: the left Orbitale with the left Tragion or the right Orbitale with the right Tragion, and being normal to the midsagittal plane;
. generating a 3D reconstruction editable surface of the individualized skeletal support wreath's (2) area on the head;
. creating the caudal and cranial boundaries of the individualized skeletal support wreath (2) surface using as references the midsagittal and Frankfort horizontal planes and at least the points, lines and linear segments (33 - 60);
whereby said points, lines and linear segments (33 - 60) include at least one of the following:
the craniometric point Glabella (G, 33)
the craniometric point Tragion (Trg, 34)
the craniometric point Orbitale (Or, 35)
the Frankfort horizontal plane (36)
the concha (37) of external ear
the middle (38) of the concha (37) on the Frankfort horizontal plane (36) the craniometric point (39) on the frontal bone contour located ten mm above Glabella (33)
the line (40) crossing the craniometric point (39) and parallel to the Frankfort horizontal plane (36)
the perpendicular (41) to the Frankfort horizontal plane (36) through the craniometric point (34)
the point (42) where the perpendicular line (41) intersects the line (40) and is the first control point of the spline curve (45)
the perpendicular (43) to the Frankfort horizontal plane (36) through the point (38)
the second control point (44) of the spline curve (45), located two mm more caudally to the point (42) and on the perpendicular (43)
the spline curve (45) consisting of four control points and one free point at its end the craniometric point Sub-Inion Right (SInR, 46)
the perpendicular (47) to Frankfort horizontal plane (36) crossing the craniometric point (46)
the third control point (48) of the spline curve (45), which is located on the perpendicular (47) and five mm above the craniometric point (46)
the craniometric point Inion (In, 49)
the fourth control point (50) of the spline curve (45), which is located five mm above the craniometric point (49) on the dorsal contour of the head
the free point (51) of the spline curve (45) at its end
the craniometric point (52) located on the frontal bone contour twenty-eight mm above the craniometric point (39)
the apex of the patient's head (53)
the perpendicular (54) to Frankfort horizontal plane (36) crossing the apex of the patient's head (53)
the point (55) on the perpendicular (54) located twenty mm below the apex of the patient's head (53)
the line (56) parallel to Frankfort horizontal plane (36) crossing the point (55)
the craniometric point (57) located on the dorsal contour of the patient's head, where the line (56) intersects the head contour
the apex of the curve of the patient's head (58) between the craniometric points (49) and (57)
the craniometric point (59) on the dorsal contour of the head located thirty mm above the craniometric point (58)
the linear segment (60) between the craniometric points (52) and (59) representing the digital cranial boundaries of the individualized skeletal support wreath (2),
. offsetting in an upward scaling to accommodate the hair volume of the patient and the thickness of the interior soft inlay (16) of the individualized skeletal support wreath (2);
. generating a solid model of the individualized skeletal support wreath (2);
. designing and positioning of the slot (12) of the individualized skeletal support wreath (2) for the connecting girder (3) in the frontal area of the individualized skeletal support wreath (2), the center of which is located on the midsagittal plane;
. designing and positioning of the reinforcement webs (18) in the frontal and lateral areas of the individualized skeletal support wreath (2);
. designing and positioning of the elliptical slits (17) for the passage of the pair of elastic straps (7) in the dorsal area of the individualized skeletal support wreath (2), determined through the angles L and R when using axilla (8) support and determined through the angles L' and R' when using groin (31) support;
. generating a 3D solid model file of the digitally designed individualized skeletal support wreath (2), such as an stl file.

6. An individualized skeletal support wreath (2) designed according to the method of claims 1-3 or manufactured according to the method of claim 4.

7. An extraoral orthopedic device (1) comprising:
. the individualized skeletal support wreath (2) of claim 6, with a frontal area and a dorsal area opposite the frontal area, whereby the said individualized skeletal support wreath (2) is configured to be placed on the periphery of the neurocranium (9) of a user;
. two elastic fixation straps (7) having support and adjustment means (26, 27) configured
to attach the straps (7) to complementary support means (17) provided on the dorsal area of the individualized skeletal support wreath (2), so that the straps (7) pass either around the shoulders and through the axillae (8) or around the top of the thighs and through the groins (31), and
to adjust the length and the pulling-power of the straps (7) between the individualized skeletal support wreath (2) and the axillae (8) or groins (31) during use;
. a mouth girder (4) provided with means (20) to attach at least one traction means (5, 10), such as an elastic band, attached to an intraoral mechanism (6, 11);
. a connecting girder (3) connecting the individualized skeletal support wreath (2) with the mouth girder (4);
. connecting and adjustment means (12, 13, 14, 15) on said individualized skeletal support wreath (2) configured to rigidly connect the connecting girder (3) at the frontal area of the individualized skeletal support wreath (2) at a proper location along its cranial part (28); and
. connecting and adjustment means (21, 22, 24, 25) on said mouth girder (4);
whereby the connecting and adjustment means (21, 22, 24, 25) on said mouth girder (4) are configured to rigidly connect the mouth girder (4) and the connecting girder (3) and adjust the relative position of the mouth girder (4) along the caudal part (30) of the connecting girder (3) and the connecting and adjustment means (12, 13, 14, 15) on said individualized skeletal support wreath (2) are configured to rigidly connect the connecting girder (3) with the said individualized skeletal support wreath (2) and adjust the relative position of the cranial part (28) of the connecting girder (3) in said individualized skeletal support wreath (2),
so that both connecting and adjustment means (12, 13, 14, 15 and 21, 22, 24, 25) can control the direction of the traction means (5, 10) during use.

8. The extraoral orthopedic device (1) according to claim 7 whereby the connecting girder (3) having a universal design of the profile of a human face contains a cranial part (28) disposed at an angle with a middle part (29) and the middle part disposed at an angle with a caudal part (30) and all of its parts (28, 29, 30) have a rectangular cross-section.

9. The extraoral orthopedic device (1) according to any one of claims 7 and 8, whereby the individualized skeletal support wreath (2) has reinforcement webs (18) configured to withstand the actions applied by the user, whereby the reinforcement webs (18) extend along an arc on the periphery of the said individualized skeletal support wreath (2) and said individualized skeletal support wreath (2) is a continuous rigid and solid ring with non-adjustable dimensions,
or the individualized skeletal support wreath (2) is a continuous rigid and solid ring with an interior soft inlay (16) in the form of a soft material pad
or in the form of an adjustable airbag inflated or deflated with the help of a valve (32) or in the form of an adjustable bag, containing air and soft material or air and liquid, inflated or deflated with the help of a valve (32).

10. The extraoral orthopedic device (1) according to any one of claims 7 to 9, whereby there is one and only one connecting girder (3) between the individualized skeletal support wreath (2) and the mouth girder (4).

11. The extraoral orthopedic device (1) according to any one of claims 7 to 10, whereby the mouth girder (4) has at least one elongated element with a mounting means (20) extended laterally from the mouth girder and configured to attach traction means (5, 10).

12. The extraoral orthopedic device (1) according to any one of claims 7 to 11, whereby the cross-section of the mouth girder (4) is orthogonal.

13. The extraoral orthopedic device (1) according to any one-of claims 7 to 12 including an intraoral mechanism (6, 11) that are connected with at least one traction means (5, 10).

14. The extraoral orthopedic device (1) according to any one of claims 7 to 13, connected by means of at least one traction means (5) to a rapid maxillary expansion mechanism (6) comprising additional components (63, 64) configured to withstand the actions applied on the maxillary teeth by the device (1).

15. The extraoral orthopedic device (1) according to any one of claims 7 to 14, that is configured to be assembled and disassembled and in which at least the connecting girder (3) and the mouth girder (4) are removable, independent elements.

## Patentansprüche

1. Ein computerimplementiertes Verfahren zum Entwerfen eines individualisierten Skelettstützkranzes (2), wobei das computerimplementierte Verfahren Folgendes umfasst:
• Erhalten einer 3D-Punktwolke eines Kopfes;
• Erzeugen eines 3D-Netzmodells des Kopfes;
• Erzeugen eines digitalen 3D-Abdruckes des Kopfes in Form einer 3D-Datei, wie beispielsweise einer STL-Datei, umfassend:
∘ Lokalisieren der Koordinaten einer Vielzahl anatomischer Punkte des Subjekts auf einem Referenzkoordinatensystem, einschließlich mindestens der folgenden Punkte: der Glabella, des linken und rechten Orbitale, des linken und rechten Tragion, des Labrale superior, des Inion (In) und der äußersten Punkte der Vorsprünge zwischen den Lineae nuchae superiores und inferiores auf den kaudo-lateralen Bereichen des Hinterhauptbeines, d. h. des Sub-Inion links (SInL) und des Sub-Inion rechts (SInR);
• Importieren der 3D-Datei in ein CAD-Softwareprogramm;
• Transformieren des gesamten Kopfes oder zumindest der kleinen Bereiche um die vorgenannten anatomischen Punkte herum und diese enthaltend in bearbeitbare Bereiche, und Definieren dieser Punkte;
• Definieren einer Mediansagittalebene, welche die Glabella, das Labrale superior und das Inion einschließt, und einer Frankfurter Horizontalebene, die mindestens eines der folgenden Punktpaare einschließt: das linke Orbitale mit dem linken Tragion oder das rechte Orbitale mit dem rechten Tragion, und die senkrecht auf der Mediansagittalebene steht;
• Erzeugen einer bearbeitbaren 3D-Rekonstruktionsoberfläche des Bereiches des individualisierten Skelettstützkranzes (2) auf dem Kopf;
• Erzeugen der kaudalen und kranialen Begrenzungen der Oberfläche des individualisierten Skelettstützkranzes (2) unter Verwendung der Mediansagittalebene und der Frankfurter Horizontalebene sowie zumindest der Punkte, Linien und Liniensegmente (33-60) als Referenzen;
wobei die Punkte, Linien und Liniensegmente (33-60) mindestens eines der Folgenden einschließen:
den kraniometrischen Punkt Glabella (G, 33)
den kraniometrischen Punkt Tragion (Trg, 34)
den kraniometrischen Punkt Orbitale (Or, 35)
die Frankfurter Horizontalebene (36)
die Concha (37) des Außenohres
die Mitte (38) der Concha (37) auf der Frankfurter Horizontalebene (36)
den kraniometrischen Punkt (39) auf der Kontur des Stirnbeines, der sich zehn mm oberhalb der Glabella (33) befindet
die Linie (40), welche den kraniometrischen Punkt (39) schneidet und parallel zur Frankfurter Horizontalebene (36) verläuft
die Senkrechte (41) zur Frankfurter Horizontalebene (36) durch den kraniometrischen Punkt (34)
den Punkt (42), an dem die senkrechte Linie (41) die Linie (40) schneidet und welcher der erste Kontrollpunkt der Spline-Kurve (45) ist
die Senkrechte (43) zur Frankfurter Horizontalebene (36) durch den Punkt (38)
den zweiten Kontrollpunkt (44) der Spline-Kurve (45), der sich zwei mm weiter kaudal zum Punkt (42) und auf der Senkrechten (43) befindet
die Spline-Kurve (45), die aus vier Kontrollpunkten und einem freien Punkt an ihrem Ende besteht
den kraniometrischen Punkt Sub-Inion rechts (SInR, 46)
die Senkrechte (47) zur Frankfurter Horizontalebene (36), welche den kraniometrischen Punkt (46) schneidet
den dritten Kontrollpunkt (48) der Spline-Kurve (45), der sich auf der Senkrechten (47) und fünf mm oberhalb des kraniometrischen Punktes (46) befindet
den kraniometrischen Punkt Inion (In, 49)
den vierten Kontrollpunkt (50) der Spline-Kurve (45), der sich fünf mm oberhalb des kraniometrischen Punktes (49) auf der dorsalen Kontur des Kopfes befindet
den freien Punkt (51) der Spline-Kurve (45) an ihrem Ende
den kraniometrischen Punkt (52), der sich auf der Kontur des Stirnbeines achtundzwanzig mm oberhalb des kraniometrischen Punktes (39) befindet
den Scheitelpunkt (Apex) des Kopfes des Patienten (53)
die Senkrechte (54) zur Frankfurter Horizontalebene (36), die den Scheitelpunkt des Kopfes des Patienten (53) schneidet
den Punkt (55) auf der Senkrechten (54), der sich zwanzig mm unterhalb des Scheitelpunktes des Kopfes des Patienten (53) befindet
die Linie (56) parallel zur Frankfurter Horizontalebene (36), welche den Punkt (55) schneidet
den kraniometrischen Punkt (57), der sich auf der dorsalen Kontur des Kopfes des Patienten befindet, wo die Linie (56) die Kopfkontur schneidet
den Scheitelpunkt der Krümmung des Kopfes des Patienten (58) zwischen den kraniometrischen Punkten (49) und (57)
den kraniometrischen Punkt (59) auf der dorsalen Kontur des Kopfes, der sich dreißig mm oberhalb des kraniometrischen Punktes (58) befindet
das Liniensegment (60) zwischen den kraniometrischen Punkten (52) und (59), welches die digitalen kranialen Begrenzungen des individualisierten Skelettstützkranzes (2) darstellt,
• Beaufschlagen mit einem Versatz (Offset) im Rahmen einer Aufwärtsskalierung, um das Haarvolumen des Patienten und die Dicke einer inneren weichen Einlage (16) des individualisierten Skelettstützkranzes (2) zu berücksichtigen;
• Erzeugen eines Volumenmodells (Solid Model) des individualisierten Skelettstützkranzes (2);
• Entwerfen und Positionieren eines Schlitzes (12) des individualisierten Skelettstützkranzes (2) für einen Verbindungssteg (3) im frontalen Bereich des individualisierten Skelettstützkranzes (2), dessen Mitte auf der Mediansagittalebene liegt;
• Entwerfen und Positionieren von Verstärkungsrippen (18) in den frontalen und lateralen Bereichen des individualisierten Skelettstützkranzes (2);
• Entwerfen und Positionieren von elliptischen Schlitzen (17) für den Durchgang eines Paares elastischer Bänder (7) im dorsalen Bereich des individualisierten Skelettstützkranzes (2), bestimmt durch die Winkel L und R bei Verwendung einer Achselstütze (8) und bestimmt durch die Winkel L' und R' bei Verwendung einer Leistenstütze (31), wobei die Verlängerungen eines Linienabschnittes, der durch die linken Achseln (AxL) und den kraniometrischen Punkt Sub-Inion links (SlnL) verläuft, und eines Linienabschnittes, der durch die rechten Achseln (AxR) und den kraniometrischen Punkt Sub-Inion rechts (SInR) verläuft, die Winkel L und R mit einer Linie bilden, die den kraniometrischen Punkt Inion (In, 49) und den Scheitelpunkt des Kopfes des Patienten (Ap, 53) kreuzt, und die Verlängerungen eines Linienabschnittes, der durch die linken Leisten (GrL) und den kraniometrischen Punkt Sub-Inion links (SlnL) verläuft, und eines Linienabschnittes, der durch die rechten Leisten (GrR) und den kraniometrischen Punkt Sub-Inion rechts (SInR) verläuft, die Winkel L' und R' mit einer Linie bilden, die den kraniometrischen Punkt Inion (In, 49) und den Scheitelpunkt des Kopfes des Patienten (Ap, 53) schneidet;
• Erzeugen einer 3D-Volumenmodelldatei des digital konstruierten individualisierten Skelettstützkranzes (2), wie beispielsweise einer STL-Datei.

2. Das computerimplementiertes Verfahren zum Entwerfen des individualisierten Skelettstützkranzes (2) nach Anspruch 1, bei dem ein digitaler 3D-Abdruck eines Kopfes unter Verwendung eines tragbaren Farb-3D-Scanners mit hybrider LED- und Infrarotlichtquelle erhalten wird.

3. Das computerimplementiertes Verfahren zum Entwerfen des individualisierten Skelettstützkranzes (2) nach Anspruch 1 oder 2, wobei der individualisierte Skelettstützkranz (2) Teil eines Schutzhelmes oder Teil eines Helmes zur Führung eines symmetrischen Schädelwachstums ist.

4. Das computerimplementiertes Verfahren zum Entwerfen des individualisierten Skelettstützkranzes (2) nach den Ansprüchen 1 bis 3, ferner umfassend die Schritte:
• Einlesen der 3D-Festkörpermodelldatei des individualisierten Skelettstützkranzes (2) in eine 3D-Druck-Slicer-Software, wobei dieses digitale Modell in Druckbefehle umgewandelt wird; und
• Herstellen des individualisierten Skelettstützkranzes (2) mittels eines 3D-Druckers.

5. Ein Computerprogramm, umfassend Befehle, die, wenn das Programm durch einen Computer ausgeführt wird, den Computer veranlassen, die folgenden Schritte des Verfahrens nach Anspruch 1 auszuführen:
• Definieren einer Mediansagittalebene, welche die Glabella, das Labrale superior und das Inion einschließt, und einer Frankfurter Horizontalebene, die mindestens eines der folgenden Punktpaare einschließt: das linke Orbitale mit dem linken Tragion oder das rechte Orbitale mit dem rechten Tragion, und die senkrecht auf der Mediansagittalebene steht;
• Erzeugen einer bearbeitbaren 3D-Rekonstruktionsoberfläche des Bereiches des individualisierten Skelettstützkranzes (2) auf dem Kopf;
• Erzeugen der kaudalen und kranialen Begrenzungen der Oberfläche des individualisierten Skelettstützkranzes (2) unter Verwendung der Mediansagittalebene und der Frankfurter Horizontalebene sowie zumindest der Punkte, Linien und Liniensegmente (33-60) als Referenzen;
wobei die Punkte, Linien und Liniensegmente (33-60) mindestens eines der Folgenden einschließen:
den kraniometrischen Punkt Glabella (G, 33)
den kraniometrischen Punkt Tragion (Trg, 34)
den kraniometrischen Punkt Orbitale (Or, 35)
die Frankfurter Horizontalebene (36)
die Concha (37) des Außenohres
die Mitte (38) der Concha (37) auf der Frankfurter Horizontalebene (36)
den kraniometrischen Punkt (39) auf der Kontur des Stirnbeines, der sich zehn mm oberhalb der Glabella (33) befindet
die Linie (40), welche den kraniometrischen Punkt (39) kreuzt und parallel zur Frankfurter Horizontalebene (36) verläuft
die Senkrechte (41) zur Frankfurter Horizontalebene (36) durch den kraniometrischen Punkt (34)
den Punkt (42), an dem die senkrechte Linie (41) die Linie (40) schneidet und welcher der erste Kontrollpunkt der Spline-Kurve (45) ist
die Senkrechte (43) zur Frankfurter Horizontalebene (36) durch den Punkt (38)
den zweiten Kontrollpunkt (44) der Spline-Kurve (45), der sich zwei mm weiter kaudal zum Punkt (42) und auf der Senkrechten (43) befindet
die Spline-Kurve (45), die aus vier Kontrollpunkten und einem freien Punkt an ihrem Ende besteht den kraniometrischen Punkt Sub-Inion rechts (SInR, 46)
die Senkrechte (47) zur Frankfurter Horizontalebene (36), welche den kraniometrischen Punkt (46) schneidet
den dritten Kontrollpunkt (48) der Spline-Kurve (45), der sich auf der Senkrechten (47) und fünf mm oberhalb des kraniometrischen Punktes (46) befindet
den kraniometrischen Punkt Inion (In, 49)
den vierten Kontrollpunkt (50) der Spline-Kurve (45), der sich fünf mm oberhalb des kraniometrischen Punktes (49) auf der dorsalen Kontur des Kopfes befindet
den freien Punkt (51) der Spline-Kurve (45) an ihrem Ende
den kraniometrischen Punkt (52), der sich auf der Kontur des Stirnbeines achtundzwanzig mm oberhalb des kraniometrischen Punktes (39) befindet
den Scheitelpunkt des Kopfes des Patienten (53)
die Senkrechte (54) zur Frankfurter Horizontalebene (36), die den Scheitelpunkt des Kopfes des Patienten (53) schneidet
den Punkt (55) auf der Senkrechten (54), der sich zwanzig mm unterhalb des Scheitelpunktes des Kopfes des Patienten (53) befindet
die Linie (56) parallel zur Frankfurter Horizontalebene (36), die den Punkt (55) schneidet
den kraniometrischen Punkt (57), der sich auf der dorsalen Kontur des Kopfes des Patienten befindet, wo die Linie (56) die Kopfkontur schneidet
den Scheitelpunkt der Krümmung des Kopfes des Patienten (58) zwischen den kraniometrischen Punkten (49) und (57)
den kraniometrischen Punkt (59) auf der dorsalen Kontur des Kopfes, der sich dreißig mm oberhalb des kraniometrischen Punktes (58) befindet
das Liniensegment (60) zwischen den kraniometrischen Punkten (52) und (59), welches die digitalen kranialen Begrenzungen des individualisierten Skelettstützkranzes (2) darstellt,
• Beaufschlagen mit einem Versatz (Offset) im Rahmen einer Aufwärtsskalierung, um das Haarvolumen des Patienten und die Dicke der inneren weichen Einlage (16) des individualisierten Skelettstützkranzes (2) zu berücksichtigen;
• Erzeugen eines Volumenmodells des individualisierten Skelettstützkranzes (2);
• Entwerfen und Positionieren des Schlitzes (12) des individualisierten Skelettstützkranzes (2) für den Verbindungssteg (3) im frontalen Bereich des individualisierten Skelettstützkranzes (2), dessen Mitte auf der Mediansagittalebene liegt;
• Entwerfen und Positionieren der Verstärkungsrippen (18) in den frontalen und lateralen Bereichen des individualisierten Skelettstützkranzes (2);
• Entwerfen und Positionieren der elliptischen Schlitze (17) für den Durchgang des Paares elastischer Bänder (7) im dorsalen Bereich des individualisierten Skelettstützkranzes (2), bestimmt durch die Winkel L und R bei Verwendung einer Achselstütze (8) und bestimmt durch die Winkel L' und R' bei Verwendung einer Leistenstütze (31);
• Erzeugen einer 3D-Festkörpermodelldatei des digital konstruierten individualisierten Skelettstützkranzes (2), wie beispielsweise einer STL-Datei.

6. Ein individualisierter Skelettstützkranz (2), entworfen nach dem Verfahren der Ansprüche 1 bis 3 oder hergestellt nach dem Verfahren von Anspruch 4.

7. Eine extraorale orthopädische Apparatur (1), umfassend:
• den individualisierten Skelettstützkranz (2) nach Anspruch 6, mit einem frontalen Bereich und einem dem frontalen Bereich gegenüberliegenden dorsalen Bereich, wobei der individualisierte Skelettstützkranz (2) so konfiguriert ist, dass er am Umfang des Neurokraniums (9) eines Benutzers platziert wird;
• zwei elastische Fixierungsbänder (7) mit Stütz- und Einstellmitteln (26, 27), die so konfiguriert sind, dass sie
die Bänder (7) an komplementären Stützmitteln (17) befestigen, die am dorsalen Bereich des individualisierten Skelettstützkranzes (2) bereitgestellt sind, so dass die Bänder (7) entweder um die Schultern und durch die Achseln (8) oder um die Oberseite der Oberschenkel und durch die Leisten (31) verlaufen, und
die Länge und die Zugkraft der Bänder (7) zwischen dem individualisierten Skelettstützkranz (2) und den Achseln (8) oder Leisten (31) während des Gebrauchs einstellen;
• einen Mundsteg (4), der mit Mitteln (20) versehen ist, um mindestens ein Zugmittel (5, 10), wie beispielsweise ein elastisches Band, zu befestigen, das an einem intraoralen Mechanismus (6, 11) angebracht ist;
• einen Verbindungssteg (3), der den individualisierten Skelettstützkranz (2) mit dem Mundsteg (4) verbindet;
• Verbindungs- und Einstellmittel (12, 13, 14, 15) an dem individualisierten Skelettstützkranz (2), die so konfiguriert sind, dass sie den Verbindungssteg (3) am frontalen Bereich des individualisierten Skelettstützkranzes (2) an einer geeigneten Stelle entlang seines kranialen Teils (28) starr verbinden; und
• Verbindungs- und Einstellmittel (21, 22, 24, 25) an dem Mundsteg (4);
• wobei die Verbindungs- und Einstellmittel (21, 22, 24, 25) an dem Mundsteg (4) so konfiguriert sind, dass sie den Mundsteg (4) und den Verbindungssteg (3) starr verbinden und die relative Position des Mundsteges (4) entlang des kaudalen Teiles (30) des Verbindungssteges (3) einstellen, und die Verbindungs- und Einstellmittel (12, 13, 14, 15) an dem individualisierten Skelettstützkranz (2) so konfiguriert sind, dass sie den Verbindungssteg (3) mit dem individualisierten Skelettstützkranz (2) starr verbinden und die relative Position des kranialen Teiles (28) des Verbindungssteges (3) in dem individualisierten Skelettstützkranz (2) einstellen,
• so dass sowohl die Verbindungs- und Einstellmittel (12, 13, 14, 15 und 21, 22, 24, 25) die Richtung des Zugmittels (5, 10) während des Gebrauchs steuern können.

8. Die extraorale orthopädische Apparatur (1) nach Anspruch 7, wobei der Verbindungssteg (3), der ein universelles Design des Profils eines menschlichen Gesichtes aufweist, einen kranialen Teil (28) enthält, der in einem Winkel zu einem mittleren Teil (29) angeordnet ist, und der mittlere Teil in einem Winkel zu einem kaudalen Teil (30) angeordnet ist, und alle seine Teile (28, 29, 30) einen rechteckigen Querschnitt aufweisen.

9. Die extraorale orthopädische Apparatur (1) nach einem der Ansprüche 7 und 8, wobei der individualisierte Skelettstützkranz (2) Verstärkungsrippen (18) aufweist, die so konfiguriert sind, dass sie den vom Benutzer aufgebrachten Kräften standhalten, wobei sich die Verstärkungsrippen (18) entlang eines Bogens am Umfang des individualisierten Skelettstützkranzes (2) erstrecken und der individualisierte Skelettstützkranz (2) ein durchgehender starrer und massiver Ring mit nicht einstellbaren Abmessungen ist,
oder der individualisierte Skelettstützkranz (2) ein durchgehender starrer und massiver Ring mit einer inneren weichen Einlage (16) in Form eines weichen Materialpolsters ist
oder in Form eines einstellbaren Airbags, der mit Hilfe eines Ventils (32) aufgeblasen oder entleert wird, ist
oder in Form eines einstellbaren Beutels bzw. Kissens, der/das Luft und weiches Material oder Luft und Flüssigkeit enthält und mit Hilfe eines Ventils (32) aufgeblasen oder entleert wird, ist.

10. Die extraorale orthopädische Apparatur (1) nach einem der Ansprüche 7 bis 9, wobei ein einziger Verbindungssteg (3) zwischen dem individualisierten Skelettstützkranz (2) und dem Mundsteg (4) vorhanden ist.

11. Die extraorale orthopädische Apparatur (1) nach einem der Ansprüche 7 bis 10, wobei der Mundsteg (4) mindestens ein langgestrecktes Element mit einem Befestigungsmittel (20) aufweist, das sich seitlich vom Mundsteg erstreckt und so konfiguriert ist, dass es Zugmittel (5, 10) befestigt.

12. Die extraorale orthopädische Apparatur (1) nach einem der Ansprüche 7 bis 11, wobei der Querschnitt des Mundsteges (4) rechteckig ist.

13. Die extraorale orthopädische Apparatur (1) nach einem der Ansprüche 7 bis 12, umfassend einen intraoralen Mechanismus (6, 11), der mit mindestens einem Zugmittel (5, 10) verbunden ist.

14. Die extraorale orthopädische Apparatur (1) nach einem der Ansprüche 7 bis 13, die mittels mindestens eines Zugmittels (5) mit einem Gaumennahtweiterungsmechanismus (6) verbunden ist, die zusätzlichen Komponenten (63, 64) umfasst, die so konfiguriert sind, dass sie den Kräften standhalten, die durch die Apparatur (1) auf die Oberkieferzähne aufgebracht werden.

15. Die extraorale orthopädische Apparatur (1) nach einem der Ansprüche 7 bis 14, die so konfiguriert ist, dass sie montiert und demontiert werden kann, und bei welcher zumindest der Verbindungssteg (3) und der Mundsteg (4) abnehmbare, unabhängige Elemente sind.

## Revendications

1. Procédé mis en œuvre par ordinateur pour la conception d'une couronne de soutien squelettique personnalisée (2), le procédé mis en œuvre par ordinateur comprenant :
- l'obtention d'un nuage de points 3D d'une tête ;
- la génération d'un modèle maillé 3D de la tête ;
- la génération d'une empreinte numérique 3D de la tête sous la forme d'un fichier 3D, tel qu'un fichier STL, comprenant :
- le repérage, dans un système de coordonnées de référence, des coordonnées d'une pluralité de points anatomiques du sujet comprenant au moins les points suivants : le point Glabelle, les points Orbitale gauche et droit, les points Tragion gauche et droit, le point Labrale supérieur, le point Inion (In), et les points les plus externes des proéminences situées entre les lignes nucales supérieure et inférieure sur les zones caudo-latérales de l'os occipital, à savoir les points Sub-Inion gauche (SInL) et Sub-Inion droit (SInR) ;
- l'importation du fichier 3D dans un logiciel de CAO ;
- la transformation en zones éditables de la tête entière ou au moins des petites zones entourant et contenant les points anatomiques susmentionnés, et la définition de ces points ;
- la définition d'un plan sagittal médian contenant le point Glabelle, le point Labrale supérieur et le point Inion, et d'un plan horizontal de Francfort contenant au moins l'une des paires de points suivantes : le point Orbitale gauche avec le point Tragion gauche ou le point Orbitale droit avec le point Tragion droit, ledit plan horizontal de Francfort étant normal au plan sagittal médian ;
- la génération d'une surface éditable de reconstruction 3D de la zone de la tête correspondant à la couronne de soutien squelettique personnalisée (2) ;
- la création des limites caudale et crâniale de la surface de la couronne de soutien squelettique personnalisée (2) en utilisant comme références le plan sagittal médian et le plan horizontal de Francfort et au moins les points, lignes et segments linéaires (33 - 60) ;
lesdits points, lignes et segments linéaires (33 - 60) comprenant au moins l'un des éléments suivants :
le point craniométrique Glabelle (G, 33)
le point craniométrique Tragion (Trg, 34)
le point craniométrique Orbitale (Or, 35)
le plan horizontal de Francfort (36)
la conque (37) de l'oreille externe
le milieu (38) de la conque (37) sur le plan horizontal de Francfort (36)
le point craniométrique (39) situé sur le contour de l'os frontal, dix mm au-dessus du point Glabelle (33)
la ligne (40) passant par le point craniométrique (39) et parallèle au plan horizontal de Francfort (36)
la perpendiculaire (41) au plan horizontal de Francfort (36) passant par le point craniométrique (34)
le point (42) où la ligne perpendiculaire (41) coupe la ligne (40), qui constitue le premier point de contrôle de la courbe spline (45)
la perpendiculaire (43) au plan horizontal de Francfort (36) passant par le point (38)
le deuxième point de contrôle (44) de la courbe spline (45), situé deux mm plus caudalement que le point (42) et sur la perpendiculaire (43)
la courbe spline (45) constituée de quatre points de contrôle et d'un point libre à son extrémité
le point craniométrique Sub-Inion droit (SInR, 46)
la perpendiculaire (47) au plan horizontal de Francfort (36) passant par le point craniométrique (46)
le troisième point de contrôle (48) de la courbe spline (45), situé sur la perpendiculaire (47) et cinq mm au-dessus du point craniométrique (46)
le point craniométrique Inion (In, 49)
le quatrième point de contrôle (50) de la courbe spline (45), situé cinq mm au-dessus du point craniométrique (49), sur le contour dorsal de la tête
le point libre (51) de la courbe spline (45) à son extrémité
le point craniométrique (52) situé sur le contour de l'os frontal, vingt-huit mm au-dessus du point craniométrique (39)
le sommet de la tête du patient (53)
la perpendiculaire (54) au plan horizontal de Francfort (36) passant par le sommet de la tête du patient (53)
le point (55) sur la perpendiculaire (54), situé vingt mm au-dessous du sommet de la tête du patient (53)
la ligne (56) parallèle au plan horizontal de Francfort (36) passant par le point (55)
le point craniométrique (57) situé sur le contour dorsal de la tête du patient, à l'endroit où la ligne (56) coupe le contour de la tête
le sommet de la courbe de la tête du patient (58) entre les points craniométriques (49) et (57)
le point craniométrique (59) sur le contour dorsal de la tête, situé trente mm au-dessus du point craniométrique (58)
le segment linéaire (60) entre les points craniométriques (52) et (59), représentant les limites crâniales numériques de la couronne de soutien squelettique personnalisée (2),
- la réalisation d'un décalage (offset) par agrandissement d'échelle afin de tenir compte du volume capillaire du patient et de l'épaisseur d'une garniture intérieure souple (16) de la couronne de soutien squelettique personnalisée (2) ;
- la génération d'un modèle volumique de la couronne de soutien squelettique personnalisée (2) ;
- la conception et le positionnement d'un logement (12) de la couronne de soutien squelettique personnalisée (2) destiné à une poutre de liaison (3), dans la zone frontale de la couronne de soutien squelettique personnalisée (2), dont le centre est situé sur le plan sagittal médian ;
- la conception et le positionnement de nervures de renfort (18) dans les zones frontale et latérales de la couronne de soutien squelettique personnalisée (2) ;
- la conception et le positionnement de fentes elliptiques (17) pour le passage d'une paire de sangles élastiques (7) dans la zone dorsale de la couronne de soutien squelettique personnalisée (2), déterminées au moyen des angles L et R en cas d'utilisation d'un appui sur les aisselles (8) et déterminées au moyen des angles L' et R' en cas d'utilisation d'un appui sur les aines (31),
dans lequel les prolongements d'un segment de droite passant par l'aisselle gauche (AxL) et le point craniométrique Sub-Inion gauche (SInL) et d'un segment de droite passant par l'aisselle droite (AxR) et le point craniométrique Sub-Inion droit (SInR) forment les angles L et R avec une droite passant par le point craniométrique Inion (In, 49) et le sommet de la tête du patient (Ap, 53), et les prolongements d'un segment de droite passant par l'aine gauche (GrL) et le point craniométrique Sub-Inion gauche (SInL) et d'un segment de droite passant par l'aine droite (GrR) et le point craniométrique Sub-Inion droit (SInR) forment les angles L' et R' avec une droite passant par le point craniométrique Inion (In, 49) et le sommet de la tête du patient (Ap, 53) ;
- la génération d'un fichier de modèle volumique 3D de la couronne de soutien squelettique personnalisée (2) conçue numériquement, tel qu'un fichier STL.

2. Procédé mis en œuvre par ordinateur pour la conception de la couronne de soutien squelettique personnalisée (2) selon la revendication 1, dans lequel une empreinte numérique 3D d'une tête est obtenue au moyen d'un scanner 3D couleur portatif à source lumineuse hybride LED et infrarouge.

3. Procédé mis en œuvre par ordinateur pour la conception de la couronne de soutien squelettique personnalisée (2) selon la revendication 1 ou 2, dans lequel la couronne de soutien squelettique personnalisée (2) fait partie d'un casque de protection ou d'un casque destiné à guider la croissance symétrique du crâne.

4. Procédé mis en œuvre par ordinateur pour la conception de la couronne de soutien squelettique personnalisée (2) selon l'une quelconque des revendications 1 à 3, comprenant en outre les étapes suivantes :
- le placement du fichier de modèle volumique 3D de la couronne de soutien squelettique personnalisée (2) dans un logiciel de découpage (slicer) pour impression 3D, transformant ce modèle numérique en instructions d'impression ; et
- la fabrication de la couronne de soutien squelettique personnalisée (2) au moyen d'une imprimante 3D.

5. Programme d'ordinateur comprenant des instructions qui, lorsque le programme est exécuté par un ordinateur, conduisent celui-ci à mettre en œuvre les étapes suivantes du procédé selon la revendication 1 :
- la définition d'un plan sagittal médian contenant le point Glabelle, le point Labrale supérieur et le point Inion, et d'un plan horizontal de Francfort contenant au moins l'une des paires de points suivantes : le point Orbitale gauche avec le point Tragion gauche ou le point Orbitale droit avec le point Tragion droit, ledit plan horizontal de Francfort étant normal au plan sagittal médian ;
- la génération d'une surface éditable de reconstruction 3D de la zone de la tête correspondant à la couronne de soutien squelettique personnalisée (2) ;
- la création des limites caudale et crâniale de la surface de la couronne de soutien squelettique personnalisée (2) en utilisant comme références le plan sagittal médian et le plan horizontal de Francfort et au moins les points, lignes et segments linéaires (33 - 60) ;
lesdits points, lignes et segments linéaires (33 - 60) comprenant au moins l'un des éléments suivants :
le point craniométrique Glabelle (G, 33)
le point craniométrique Tragion (Trg, 34)
le point craniométrique Orbitale (Or, 35)
le plan horizontal de Francfort (36)
la conque (37) de l'oreille externe
le milieu (38) de la conque (37) sur le plan horizontal de Francfort (36)
le point craniométrique (39) situé sur le contour de l'os frontal, dix mm au-dessus du point Glabelle (33)
la ligne (40) passant par le point craniométrique (39) et parallèle au plan horizontal de Francfort (36)
la perpendiculaire (41) au plan horizontal de Francfort (36) passant par le point craniométrique (34)
le point (42) où la ligne perpendiculaire (41) coupe la ligne (40), qui constitue le premier point de contrôle de la courbe spline (45)
la perpendiculaire (43) au plan horizontal de Francfort (36) passant par le point (38)
le deuxième point de contrôle (44) de la courbe spline (45), situé deux mm plus caudalement que le point (42) et sur la perpendiculaire (43)
la courbe spline (45) constituée de quatre points de contrôle et d'un point libre à son extrémité
le point craniométrique Sub-Inion droit (SInR, 46)
la perpendiculaire (47) au plan horizontal de Francfort (36) passant par le point craniométrique (46)
le troisième point de contrôle (48) de la courbe spline (45), situé sur la perpendiculaire (47) et cinq mm au-dessus du point craniométrique (46)
le point craniométrique Inion (In, 49)
le quatrième point de contrôle (50) de la courbe spline (45), situé cinq mm au-dessus du point craniométrique (49), sur le contour dorsal de la tête
le point libre (51) de la courbe spline (45) à son extrémité
le point craniométrique (52) situé sur le contour de l'os frontal, vingt-huit mm au-dessus du point craniométrique (39)
le sommet de la tête du patient (53)
la perpendiculaire (54) au plan horizontal de Francfort (36) passant par le sommet de la tête du patient (53)
le point (55) sur la perpendiculaire (54), situé vingt mm au-dessous du sommet de la tête du patient (53)
la ligne (56) parallèle au plan horizontal de Francfort (36) passant par le point (55)
le point craniométrique (57) situé sur le contour dorsal de la tête du patient, à l'endroit où la ligne (56) coupe le contour de la tête
le sommet de la courbe de la tête du patient (58) entre les points craniométriques (49) et (57)
le point craniométrique (59) sur le contour dorsal de la tête, situé trente mm au-dessus du point craniométrique (58)
le segment linéaire (60) entre les points craniométriques (52) et (59), représentant les limites crâniales numériques de la couronne de soutien squelettique personnalisée (2),
- la réalisation d'un décalage (offset) par agrandissement d'échelle afin de tenir compte du volume capillaire du patient et de l'épaisseur de la garniture intérieure souple (16) de la couronne de soutien squelettique personnalisée (2) ;
- la génération d'un modèle volumique de la couronne de soutien squelettique personnalisée (2) ;
- la conception et le positionnement du logement (12) de la couronne de soutien squelettique personnalisée (2) destiné à la poutre de liaison (3), dans la zone frontale de la couronne de soutien squelettique personnalisée (2), dont le centre est situé sur le plan sagittal médian ;
- la conception et le positionnement des nervures de renfort (18) dans les zones frontale et latérales de la couronne de soutien squelettique personnalisée (2) ;
- la conception et le positionnement des fentes elliptiques (17) pour le passage de la paire de sangles élastiques (7) dans la zone dorsale de la couronne de soutien squelettique personnalisée (2), déterminées au moyen des angles L et R en cas d'utilisation d'un appui sur les aisselles (8) et déterminées au moyen des angles L' et R' en cas d'utilisation d'un appui sur les aines (31) ;
- la génération d'un fichier de modèle volumique 3D de la couronne de soutien squelettique personnalisée (2) conçue numériquement, tel qu'un fichier STL.

6. Couronne de soutien squelettique personnalisée (2) conçue selon le procédé de l'une quelconque des revendications 1 à 3 ou fabriquée selon le procédé de la revendication 4.

7. Dispositif orthopédique extra-oral (1) comprenant :
- la couronne de soutien squelettique personnalisée (2) selon la revendication 6, présentant une zone frontale et une zone dorsale opposée à la zone frontale, ladite couronne de soutien squelettique personnalisée (2) étant configurée pour être placée sur la périphérie du neurocrâne (9) d'un utilisateur ;
- deux sangles de fixation élastiques (7) comportant des moyens de support et de réglage (26, 27) configurés pour fixer les sangles (7) à des moyens de support complémentaires (17) prévus sur la zone dorsale de la couronne de soutien squelettique personnalisée (2), de sorte que les sangles (7) passent soit autour des épaules et par les aisselles (8), soit autour du haut des cuisses et par les aines (31), et
pour régler la longueur et la force de traction des sangles (7) entre la couronne de soutien squelettique personnalisée (2) et les aisselles (8) ou les aines (31) en cours d'utilisation ;
- une poutre buccale (4) pourvue de moyens (20) pour fixer au moins un moyen de traction (5, 10), tel qu'un élastique, fixé à un mécanisme intra-oral (6, 11) ;
- une poutre de liaison (3) reliant la couronne de soutien squelettique personnalisée (2) à la poutre buccale (4) ;
- des moyens de liaison et de réglage (12, 13, 14, 15) sur ladite couronne de soutien squelettique personnalisée (2), configurés pour relier rigidement la poutre de liaison (3) au niveau de la zone frontale de la couronne de soutien squelettique personnalisée (2) à un emplacement approprié le long de sa partie crâniale (28) ; et
- des moyens de liaison et de réglage (21, 22, 24, 25) sur ladite poutre buccale (4) ;
les moyens de liaison et de réglage (21, 22, 24, 25) sur ladite poutre buccale (4) étant configurés pour relier rigidement la poutre buccale (4) et la poutre de liaison (3) et pour régler la position relative de la poutre buccale (4) le long de la partie caudale (30) de la poutre de liaison (3), et les moyens de liaison et de réglage (12, 13, 14, 15) sur ladite couronne de soutien squelettique personnalisée (2) étant configurés pour relier rigidement la poutre de liaison (3) à ladite couronne de soutien squelettique personnalisée (2) et pour régler la position relative de la partie crâniale (28) de la poutre de liaison (3) dans ladite couronne de soutien squelettique personnalisée (2),
de sorte que les deux ensembles de moyens de liaison et de réglage (12, 13, 14, 15 et 21, 22, 24, 25) permettent de contrôler la direction des moyens de traction (5, 10) en cours d'utilisation.

8. Dispositif orthopédique extra-oral (1) selon la revendication 7, dans lequel la poutre de liaison (3), présentant une conception universelle du profil d'un visage humain, comporte une partie crâniale (28) disposée selon un angle par rapport à une partie médiane (29), la partie médiane étant disposée selon un angle par rapport à une partie caudale (30), et toutes ses parties (28, 29, 30) présentent une section transversale rectangulaire.

9. Dispositif orthopédique extra-oral (1) selon l'une quelconque des revendications 7 et 8, dans lequel la couronne de soutien squelettique personnalisée (2) comporte des nervures de renfort (18) configurées pour résister aux efforts exercés par l'utilisateur, les nervures de renfort (18) s'étendant selon un arc sur la périphérie de ladite couronne de soutien squelettique personnalisée (2), et ladite couronne de soutien squelettique personnalisée (2) est un anneau continu, rigide et plein, de dimensions non réglables,
ou la couronne de soutien squelettique personnalisée (2) est un anneau continu, rigide et plein, muni d'une garniture intérieure souple (16) sous la forme d'un coussinet en matériau souple
ou sous la forme d'un coussin d'air réglable, gonflé ou dégonflé à l'aide d'une valve (32)
ou sous la forme d'une poche réglable, contenant de l'air et un matériau souple ou de l'air et un liquide, gonflée ou dégonflée à l'aide d'une valve (32).

10. Dispositif orthopédique extra-oral (1) selon l'une quelconque des revendications 7 à 9, dans lequel il existe une seule et unique poutre de liaison (3) entre la couronne de soutien squelettique personnalisée (2) et la poutre buccale (4).

11. Dispositif orthopédique extra-oral (1) selon l'une quelconque des revendications 7 à 10, dans lequel la poutre buccale (4) comporte au moins un élément allongé muni d'un moyen de montage (20), s'étendant latéralement depuis la poutre buccale et configuré pour fixer des moyens de traction (5, 10).

12. Dispositif orthopédique extra-oral (1) selon l'une quelconque des revendications 7 à 11, dans lequel la section transversale de la poutre buccale (4) est orthogonale.

13. Dispositif orthopédique extra-oral (1) selon l'une quelconque des revendications 7 à 12, comprenant un mécanisme intra-oral (6, 11) relié à au moins un moyen de traction (5, 10).

14. Dispositif orthopédique extra-oral (1) selon l'une quelconque des revendications 7 à 13, relié, au moyen d'au moins un moyen de traction (5), à un mécanisme d'expansion maxillaire rapide (6) comprenant des composants supplémentaires (63, 64) configurés pour résister aux efforts exercés sur les dents maxillaires par le dispositif (1).

15. Dispositif orthopédique extra-oral (1) selon l'une quelconque des revendications 7 à 14, configuré pour être assemblé et désassemblé, et dans lequel au moins la poutre de liaison (3) et la poutre buccale (4) sont des éléments amovibles indépendants.
